(19) Europäisches Patentamt
European Patent Office
Office européen des brevets

(11) EP 0 564 409 B1

(12) # EUROPÄISCHE PATENTSCHRIFT

(45) Veröffentlichungstag und Bekanntmachung des Hinweises auf die Patenterteilung:
**19.01.2000 Patentblatt 2000/03**

(51) Int. Cl.7: **C07D 401/04**, C07D 403/04, C07D 401/14, A61K 31/505

(21) Anmeldenummer: **93810219.1**

(22) Anmeldetag: **25.03.1993**

(54) **Pyrimidinderivate und Verfahren zu ihrer Herstellung**
Pyrimidin derivatives and process for their preparation
Dérivés de pyrimidine et procédé pour leur préparation

(84) Benannte Vertragsstaaten:
**AT BE CH DE DK ES FR GB GR IE IT LI LU NL PT SE**

(30) Priorität: **03.04.1992 CH 108392**

(43) Veröffentlichungstag der Anmeldung:
**06.10.1993 Patentblatt 1993/40**

(73) Patentinhaber:
- **Novartis AG**
**4058 Basel (CH)**
Benannte Vertragsstaaten:
**BE CH DE DK ES FR GB GR IE IT LI LU NL PT SE**
- **Novartis-Erfindungen Verwaltungsgesellschaft m.b.H.**
**1235 Wien (AT)**
Benannte Vertragsstaaten:
**AT**

(72) Erfinder: **Zimmermann, Jürg, Dr.**
**CH-4313 Möhlin (CH)**

(56) Entgegenhaltungen:
**EP-A- 0 233 461**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 564 409 B1

## Beschreibung

Die Erfindung betrifft N-Phenyl-2-pyrimidinamin-derivate, Verfahren zu ihrer Herstellung, Arzneimittel, enthaltend diese Verbindungen, und deren Verwendung zur Herstellung von pharmazeutischen Präparaten zur therapeutischen Behandlung von Warmblütern.

Die Erfindung betrifft N-Phenyl-2-pyrimidinamin-derivate der Formel I,

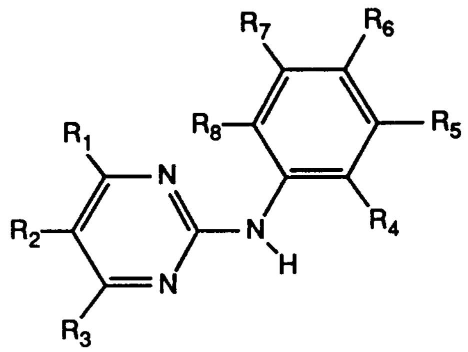

(I)

worin $R_1$ 4-Pyrazinyl, 1-Methyl-1H-pyrrolyl, durch Amino oder Aminoniederalkyl substituiertes Phenyl, worin die Aminogruppe jeweils frei, alkyliert oder acyliert ist, an einem Fünfringkohlenstoffatom gebundenes 1H-Indolyl oder 1H-Imidazolyl, oder an einem Ringkohlenstoffatom gebundenes, unsubstituiertes oder durch Niederalkyl substituiertes Pyridyl, welches am Stickstoff unsubstituiert oder durch Sauerstoff substituiert ist, bedeutet, $R_2$ und $R_3$ unabhängig voneinander je Wasserstoff oder Niederalkyl bedeuten, einer oder zwei der Reste $R_4$, $R_5$, $R_6$, $R_7$ und $R_8$ je Nitro, fluorsubstituiertes Niederalkoxy oder einen Rest der Formel II bedeuten,

$$-N(R_9)-C(=X)-(Y)_n-R_{10} \qquad (II)$$

worin $R_9$ für Wasserstoff oder Niederalkyl, X für Oxo, Thio, Imino, N-Niederalkyl-imino, Hydroximino oder O-Niederalkylhydroximino, Y für Sauerstoff oder die Gruppe NH, n für 0 oder 1 und $R_{10}$ für einen aliphatischen Rest mit mindestens 5 C-Atomen oder für einen aromatischen, aromatisch-aliphatischen, cycloaliphatischen, cycloaliphatisch-aliphatischen, heterocyclischen oder heterocyclisch-aliphatischen Rest steht, und die übrigen der Reste $R_4$, $R_5$, $R_6$, $R_7$ und $R_8$ unabhängig voneinander je Wasserstoff, Niederalkyl, welches unsubstituiert oder durch freies oder alkyliertes Amino, Piperazinyl, Piperidinyl, Pyrrolidinyl oder Morpholinyl substituiert ist, Niederalkanoyl, Trifluormethyl, freies, verethertes oder verestertes Hydroxy, freies, alkyliertes oder acyliertes Amino oder freies oder verestertes Carboxy bedeuten, und Salze von solchen Verbindungen mit mindestens einer salzbildenden Gruppe.

1-Methyl-1H-pyrrolyl ist vorzugsweise 1-Methyl-1H-pyrrol-2-yl oder 1-Methyl-1H-pyrrol-3-yl.

Durch Amino oder Aminoniederalkyl substituiertes Phenyl $R_1$, worin die Aminogruppe jeweils frei, alkyliert oder acyliert ist, ist in beliebiger Stellung (ortho, meta oder para) substituiertes Phenyl, worin eine alkylierte Aminogruppe vorzugsweise Mono-oder Diniederalkylamino, z.B. Dimethylamino, ist und der Niederalkyl-Teil von Aminoniederalkyl, vorzugsweise lineares $C_1$-$C_3$-Alkyl, wie insbesondere Methyl oder Ethyl, ist.

An einem Fünfringkohlenstoffatom gebundenes 1H-Indolyl ist 1H-Indol-2-yl oder 1H-Indol-3-yl.

An einem Ringkohlenstoffatom gebundenes, unsubstituiertes oder durch Niederalkyl substituiertes Pyridyl ist durch Niederalkyl substituiertes oder vorzugsweise unsubstituiertes 2-, 4- oder vorzugsweise 3-Pyridyl, z.B. 3-Pyridyl, 2-Methyl-3-pyridyl oder 4-Methyl-3-pyridyl. Pyridyl, welches am Stickstoff durch Sauerstoff substituiert ist, ist ein vom Pyridin-N-oxid abgeleiteter Rest, d.h. N-Oxido-pyridyl.

Fluorsubstituiertes Niederalkoxy ist Niederalkoxy, welches mindestens einen, vorzugsweise aber mehrere Fluorsubstituenten trägt, insbesondere Trifluormethoxy der 1,1,2,2-Tetrafluor-ethoxy.

Wenn X für Oxo, Thio, Imino, N-Niederalkyl-imino, Hydroximino oder O-Niederalkylhydroximino steht, stellt die Gruppe C=X in der genannten Reihenfolge die Reste C=O, C=S, C=N-H, C=N-Niederalkyl, C=N-OH beziehungsweise C=N-O-Niederalkyl dar. Vorzugsweise steht X für Oxo.

Vorzugsweise steht n für 0, d.h. die Gruppe Y ist nicht vorhanden.

Y, falls vorhanden, steht vorzugsweise für die Gruppe NH.

Das Präfix "Nieder-" bezeichnet im Rahmen dieses Textes Reste bis und mit 7, vorzugsweise bis und mit 4 C-Atomen.

Niederalkyl $R_1$, $R_2$, $R_3$ und $R_9$ ist vorzugsweise Methyl oder Ethyl.

Ein aliphatischer Rest $R_{10}$ mit mindestens 5 C-Atomen hat vorzugsweise nicht mehr als 22 C-Atome, in der Regel nicht mehr als 10 C-Atome und ist ein solcher substituierter oder vorzugsweise unsubstituierter aliphatischer Kohlenwasserstoffrest, das heisst ein solcher substituierter oder vorzugsweise unsubstituierter Alkinyl-, Alkenyl- oder vorzugsweise Alkylrest, wie $C_5$-$C_7$-Alkyl, z.B. n-Pentyl. Ein aromatischer Rest $R_{10}$ hat bis zu 20 C-Atome und ist unsubstituiert oder substituiert, z.B. jeweils unsubstituiertes oder substituiertes Naphthyl, wie insbesondere 2-Naphthyl, oder vorzugsweise Phenyl, wobei die Substituenten vorzugsweise ausgewählt sind aus Cyano, unsubstituiertem oder durch Hydroxy, Amino oder 4-Methyl-piperazinyl substituiertem Niederalkyl, wie insbesondere Methyl, aus Trifluormethyl, freiem, verethertem oder verestertem Hydroxy, freiem, alkyliertem oder acyliertem Amino und aus freiem oder verestertem Carboxy. In einem aromatisch-aliphatischen Rest $R_{10}$ ist der aromatische Teil wie vorstehend definiert und der aliphatische Teil ist vorzugsweise Niederalkyl, wie insbesondere $C_1$-$C_2$-Alkyl, welches substituiert oder vorzugsweise unsubstituiert ist, z.B. Benzyl. Ein cycloaliphatischer Rest $R_{10}$ hat insbesondere bis zu 30, hauptsächlich bis zu 20 und in erster Linie bis zu 10 C-Atome, ist mono- oder polycyclisch und substituiert oder vorzugsweise unsubstituiert, z.B. ein solcher, inbesondere 5- oder 6gliedriger Cycloalkylrest, wie vorzugsweise Cyclohexyl. In einem cycloaliphatisch-aliphatischen Rest Rest $R_{10}$ ist der cycloaliphatische Teil wie vorstehend definiert und der aliphatische Teil ist vorzugsweise Niederalkyl, wie insbesondere $C_1$-$C_2$-Alkyl, welches substituiert oder vorzugsweise unsubstituiert ist. Ein heterocyclischer Rest $R_{10}$ enthält insbesondere bis zu 20 C-Atome und ist vorzugsweise ein gesättigter oder ungesättigter monocyclischer Rest mit 5 oder 6 Ringgliedern und 1-3 Heteroatomen, welche vorzugsweise aus Stickstoff, Sauerstoff und Schwefel ausgewählt sind, insbesondere z.B. Thienyl oder 2-, 3- oder 4-Pyridyl, oder ein bi-oder tricyclischer Rest, worin z.B. an den genannten monocyclischen Rest ein oder zwei Benzolreste ankondensiert (annelliert) sind. In einem heterocyclisch-aliphatischen Rest Rest $R_{10}$ ist der heterocyclische Teil wie vorstehend definiert und der aliphatische Teil ist vorzugsweise Niederalkyl, wie insbesondere $C_1$-$C_2$-Alkyl, welches substituiert oder vorzugsweise unsubstituiert ist.

Verethertes Hydroxy ist vorzugsweise Niederalkoxy. Verestertes Hydroxy ist vorzugsweise mit einer organischen Carbonsäure, wie einer Niederalkansäure, oder einer Mineralsäure, wie einer Halogenwasserstoffsäure, verestertes Hydroxy, z.B. Niederalkanoyloxy oder insbesondere Halogen, wie Iod, Brom oder besonders Fluor oder Chlor.

Alkyliertes Amino ist z.B. Niederalkylamino, wie Methylamino, oder Dinierderalkylamino, wie Dimethylamino. Acyliertes Amino ist z.B. Niederalkanoylamino oder Benzoylamino.

Verestertes Carboxy ist z.B. Niederalkoxycarbonyl, wie Methoxycarbonyl.

Ein substituierter Phenylrest kann bis zu 5 Substituenten, wie z.B. Fluor, tragen, ist aber insbesondere bei grösseren Substituenten in der Regel nur 1-3fach substituiert. Als Beispiele für substituiertes Phenyl seien 4-Chlorphenyl, Pentafluor-phenyl, 2-Carboxy-phenyl, 2-Methoxy-phenyl, 4-Fluor-phenyl, 4-Cyano-phenyl und 4-Methyl-phenyl hervorgehoben.

Salzbildende Gruppen in einer Verbindung der Formel I sind Gruppen oder Reste mit basischen oder sauren Eigenschaften. Verbindungen mit mindestens einer barischen Gruppe oder mindestens einem basischen Rest, z. B. einer freien Aminogruppe, einem Pyrazinylrest oder einem Pyridylrest können Säureadditionssalze bilden, z.B. mit anorganischen Säuren, wie Salzsäure, Schwefelsäure oder einer Phosphorsäure, oder mit geeigneten organischen Carbon- oder Sulfonsäuren, z.B. aliphatischen Mono- oder Dicarbonsäuren, wie Trifluoressigsäure, Essigsäure, Propionsäure, Glykolsäure, Bernsteinsäure, Maleinsäure, Fumarsäure, Hydroxymaleinsäure, Aepfelsäure, Weinsäure, Zitronensäure, Oxalsäure oder Aminosäuren, wie Arginin oder Lysin, aromatischen Carbonsäuren, wie Benzoesäure, 2-Phenoxy-benzoesäure, 2-Acetoxy-benzoesäure, Salicylsäure, 4-Aminosalicylsäure, aromatisch-aliphatischen Carbonsäuren, wie Mandelsäure oder Zimtsäure, heteroaromatischen Carbonsäuren, wie Nicotinsäure oder Isonicotinsäure, aliphatischen Sulfonsäuren, wie Methan-, Ethan- oder 2-Hydroxy-ethan-sulfonsäure, oder aromatischen Sulfonsäuren, z.B. Benzol-, p-Toluol- oder Naphthalin-2-sulfonsäure. Bei Anwesenheit von mehreren basischen Gruppen können Mono- oder Polysäureadditionssalze gebildet werden.

Verbindungen der Formel I mit sauren Gruppen, z.B. einer freien Carboxylgruppe im Rest $R_{10}$, können Metall- oder Ammoniumsalze, wie Alkalimetall- oder Erdalkalimetallsalze bilden, z.B. Natrium-, Kalium-, Magnesium- oder Calciumsalze oder Ammoniumsalze mit Ammoniak oder geeigeten organischen Aminen, wie tertiären Monoaminen, z.B. Triethylamin oder Tri-(2-hydroxyethyl)-amin, oder heterocyclischen Basen, z.B. N-Ethyl-piperidin oder N,N'-Dimethylpiperazin.

Verbindungen der Formel I, die sowohl saure als auch basische Gruppen besitzen, können innere Salze bilden.

Zur Isolierung oder Reinigung sowie bei den als Zwischenprodukt weiterverwendeten Verbindungen können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Infolge der engen Beziehung zwischen den neuen Verbindungen in freier Form und in Form ihrer Salze, inkl. auch solcher Salze, die als Zwischenprodukte, z.B. bei der Reinigung der neuen Verbindungen oder zu ihrer Identifikation verwendet werden können, sind vorausgehend und nachfolgend unter den freien Verbindungen sinn- und zweckgemäss gegebenenfalls auch die entsprechenden Salze zu verstehen.

Die Verbindungen der Formel I besitzen wertvolle pharmakologische Eigenschaften und können z.B. als Antitumormittel und als Mittel gegen Atherosklerose verwendet werden.

Die Phosphorylierung von Proteinen ist seit langem als ein wesentlicher Schritt bei der Differenzierung und Vermehrung von Zellen bekannt. Die Phosphorylierung wird durch Proteinkinasen katalysiert, die man in Serin/Threonin-Kinasen und Tyrosin-Kinasen unterteilt. Zu den Serin/Threonin-Kinasen gehört die Proteinkinase C, zu den Tyrosin-Kinasen gehört die PDGF (Platelet-derived Growth Factor)-Rezeptor-Tyrosin-Kinase.

Die Verbindungen der Formel I, worin $R_4$ und $R_8$ für Wasserstoff stehen, hemmen selektiv das Enzym Proteinkinase C.

Die von Phospholipiden und Calcium abhängige Proteinkinase C kommt innerhalb der Zelle in mehreren Spezies (Verteilung der Spezies gewebespezifisch) vor und beteiligt sich an verschiedenen fundamentalen Vorgängen, wie Signalübertragung, Proliferation und Differenzierung, sowie auch Ausschüttung von Hormonen und Neurotransmittern. Die Aktivierung dieses Enzyms erfolgt entweder durch eine über Rezeptoren vermittelte Hydrolyse von Phospholipiden der Zellmembran oder durch eine direkte Interaktion mit gewissen Tumor-fördernden Wirkstoffen. Zelluläre Funktionen, die mit Hilfe der Proteinkinase C gesteuert werden, können durch die Modulation der Enzymaktivität von Proteinkinase C beeinflusst werden.

Zur Bestimmung der Proteinkinase-C-Hemmwirkung verwendet man Proteinkinase C aus Schweinehirn, welche gemäss der von T. Uchida und C.R. Filburn in J. Biol. Chem. 259, 12311-4 (1984) beschriebenen Verfahrensweise gereinigt wird. Die Bestimmung der Proteinkinase-C-Hemmwirkung der Verbindungen der Formel I erfolgt nach der Methodik von D. Fabro et al., Arch. Biochem. Biophys. 239, 102-111 (1985). In diesem Test hemmen die Verbindungen der Formel I die Proteinkinase C bereits bei einer Konzentration $IC_{50}$ zwischen etwa 0,1 und 10 µMol/Liter, insbesondere zwischen etwa 0,05 und 5 µMol/Liter. Demgegenüber hemmen die Verbindungen der Formel I andere Enzyme, z.B. Proteinkinase A, Protein-Phosphorylase-Kinase und bestimmte Typen von Protein-Tyrosin-Kinase, z.B. die Protein-Tyrosin-Kinase des EGF (Epidermal Growth Factor)-Rezeptors, erst bei einer weitaus, z.B. 100fach höheren Konzentration. Dies zeigt die Selektivität der Verbindungen der Formel I. Im Hinblick auf eine Verringerung unerwünschter Nebenwirkungen, ist es wichtig, dass die Proteinkinase-C-Hemmstoffe möglichst selektiv sind, also unter anderen andere Enzyme möglichst wenig beeinflussen, insbesondere wenn die Beeinflussung der Aktivität jener anderen Enzyme im Hinblick auf die zu behandelnde Krankheit keinen gleichwirkenden oder synergistischen Effekt hat.

Aufgrund ihrer Hemmwirkung gegenüber Proteinkinase C können die Verbindungen der Formel I, worin $R_4$ und $R_8$ Wasserstoff stehen, und ihre pharmazeutisch verwendbaren Salze als tumorhemmende, immunomodulierende und antibakterielle Wirkstoffe, ferner als Mittel gegen Atherosklerose, die Immunschwächekrankheit AIDS, sowie Krankheiten des kardiovaskulären Systems und des zentralen Nervensystems, verwendet werden.

Wie bereits aufgrund der obengeschilderten Hemmwirkung auf Proteinkinase C erwartet werden kann, weisen die Verbindungen der Formel I, worin $R_4$ und $R_8$ für Wasserstoff stehen, und ihre pharmazeutisch verwendbaren Salze antiproliferative Eigenschaften auf, die sich in folgendem anderen Versuch direkt demonstrieren lassen: Dabei wird die Hemmwirkung der Verbindungen der Formel I auf das Wachstum von menschlichen T24 Blasenkarzinomzellen bestimmt. Diese Zellen werden in "Eagle's" minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen (1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (G/V) wässriger Glutaraldehydlösung fixiert, mit Wasser gewaschen und mit 0,05%iger (Gewicht/Volumen) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportional ist, mit einem Photometer (Titertek multiskan) bei 665 nm gemessen. Die $IC_{50}$-Werte werden mit einem Computersystem unter Verwendung der Formel

$$\frac{OD_{665}(\text{Test}) \text{ minus } OD_{665}(\text{Anfang})}{OD_{665}(\text{Kontrolle}) \text{ minus } OD_{665}(\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. Die so ermittelten $IC_{50}$-Werte liegen für die Verbindungen der Formel I zwischen etwa 0,1 und 10 µMol/Liter.

Aufgrund der beschriebenen Eigenschaften können die Verbindungen der Formel I, worin $R_4$ und $R_8$ für Wasserstoff stehen, insbesondere als tumorhemmende Wirkstoffe verwendet werden, z.B zur Therapie von Tumoren der Blase. Ausserdem kommen sie für die oben für Proteinkinase C Modulatoren genannten weiteren Anwendungen in Betracht und können insbesondere zur Behandlung von Krankheiten verwendet werden, die auf eine Hemmung der Proteinkinase C ansprechen.

Die Verbindungen der Formel I, worin $R_4$ und $R_8$ für Wasserstoff stehen, hemmen zum Teil nicht nur die Proteinkinase C, sondern bereits bei einer Konzentration $IC_{50}$ zwischen etwa 0,01 und 5 μMol/Liter, insbesondere zwischen etwa 0,05 und 1 μMol/Liter, auch bestimmte Tyrosin-Kinasen, wie insbesondere die PDGF-Rezeptor-Kinase oder die abl-Kinase, z.B. die v-abl-Kinase. Verbindungen der Formel I, worin mindestens einer der Reste $R_4$ und $R_8$ von Wasserstoff verschieden ist, und z.B. für Niederalkyl, wie Methyl, steht, sind besonders selektiv für die obengenannten PDGF-Rezeptor und abl-Tyrosin-Kinasen und hemmen Proteinkinase C praktisch nicht.

PDGF (Platelet-derived Growth Factor) ist ein sehr häng vorkommender Wachstumsfaktor, der eine wichtige Rolle sowohl beim normalen Wachstum als auch bei der pathologischen Zellvermehrung spielt, wie bei der Karzinogenese und bei Erkrankungen der glasen Muskelzellen von Blutgefässen, z.B. bei der Atherosklerose und der Thrombose.

Die Hemmung der Proteinkinase C und der PDGF-Rezeptor-Kinase wirkt in diesem Sinne quasi synergistisch in die gleiche Richtung im Hinblick auf die Regulierung des Zellwachstums.

Die Hemmung der PDGF-stimulierten Rezeptor-Tyrosinkinaseaktivität in vitro wird in PDGF Rezeptor Immunkomplexen von BALB/c 3T3 Zellen gemessen, analog wie beschrieben von E. Andrejauskas-Buchdunger und U. Regenass in Cancer Research 52, 5353-5358 (1992). Die oben näher bezeichneten Verbindungen der Formel I hemmen die PDGF-abhängige zellfreie Rezeptorphosphorylierung bei Konzentrationen von 0,005-5 μMol/Liter, insbesondere 0,01-1,0, hauptsächlich 0,01-0,1 μMol/Liter. Die Hemmung der PDGF-Rezeptor-Tyrosinkinase in der intakten Zelle wird mittels Western Plot Analyse nachgewiesen, ebenfalls analog wie beschrieben von E. Andrejauskas-Buchdunger und U. Regenass in Cancer Research 52, 5353-5358 (1992). In diesem Test wird die Hemmung der Ligand-stimulierten PDGF-Rezeptor-Autophosphorylierung in BALB/c Mauszellen mit Hilfe von Anti-Phosphotyrosin-Antikörper gemessen. Die oben näher bezeichneten Verbindungen der Formel I hemmen die Tyrosinkinase Aktivität des PDGF-Rezeptors bei Konzentrationen von 0,005-5 μMol/Liter, insbesondere 0,01-1,0, hauptsächlich 0,01-0,1 μMol/Liter. Diese Verbindungen hemmen ausserdem in Konzentrationen unterhalb von 1,0 μMol/Liter auch das Zellwachstum einer PDGF-abhängigen Zelllinie, nämlich der BALB/c 3T3 Mausfibroblasten.

Die obengenannte Hemmung der v-abl-Tyrosinkinase wird nach den Methoden von N. Lydon et al., Oncogene Research 5, 161-173 (1990) und J. F. Geissler et al., Cancer Research 52, 4492-4498 (1992) bestimmt. Dabei verwendet man [$Val^5$]-Angiotensin II und [$\gamma$-$^{32}P$]-ATP als Substrate.

Aufgrund der beschriebenen Eigenschaften können Verbindungen der Formel I nicht nur als tumorhemmende Wirkstoffe, sondern auch als Mittel gegen nicht-maligne proliferative Erkrankungen, wie Atherosklerose, Thrombose, Psioriasis, Sklerodermie und Fibrose verwendet werden. Ausserdem kommen sie für die oben für Proteinkinase C Modulatoren genannten weiteren Anwendungen in Betracht und können insbesondere zur Behandlung von Krankheiten verwendet werden, die auf eine Hemmung der PDGF-Rezeptor-Kinase ansprechen.

Ausserdem verhindern die Verbindungen der Formel I die Bildung von Resistenz (multi-drug resistance) bei der Krebstherapie mit anderen Chemotherapeutika oder heben eine bereits gegenüber anderen Chemotherapeutika vorhandene Resistenz auf.

Bevorzugt sind Verbindungen der Formel I, worin einer oder zwei der Reste $R_4$, $R_5$, $R_6$, $R_7$ und $R_8$ je Nitro oder einen Rest der Formel II bedeuten, worin $R_9$ für Wasserstoff oder Niederalkyl, X für Oxo, Thio, Imino, N-Niederalkyl-imino, Hydroximino oder O-Niederalkyl-hydroximino, Y für Sauerstoff oder die Gruppe NH, n für 0 oder 1 und $R_{10}$ für einen aliphatischen Rest mit mindestens 5 C-Atomen oder für einen aromatischen, aromatischaliphatischen, cycloaliphatischen, cycloaliphatisch-aliphatischen, heterocyclischen oder heterocyclisch-aliphatischen Rest steht, und die übrigen der Reste $R_4$, $R_5$, $R_6$, $R_7$ und $R_8$ unabhängig voneinander je Wasserstoff, Niederalkyl, welches unsubstituiert oder durch freies oder alkyliertes Amino, Piperazinyl, Piperidinyl, Pyrrolidinyl oder Morpholinyl substituiert ist, Niederalkanoyl, Trifluormethyl, freies, verethertes oder verestertes Hydroxy, freies, alkyliertes oder acyliertes Amino oder freies oder verestertes Carboxy bedeuten, und die übrigen Substituenten die obengenannten Bedeutungen haben, und Salze von solchen Verbindungen mit mindestens einer salzbildenden Gruppe.

Bevorzugt sind insbesondere Verbindungen der Formel I, worin $R_1$ 4-Pyrazinyl, 1-Methyl-1H-pyrrolyl, durch Amino oder Aminoniederalkyl substituiertes Phenyl, worin die Aminogruppe jeweils frei, durch ein oder zwei Niederalkylreste alkyliert oder durch Niederalkanoyl oder Benzoyl acyliert ist, an einem Fünfringkohlenstoffatom gebundenes 1H-Indolyl oder 1H-Imidazolyl, oder an einem Ringkohlenstoffatom gebundenes,unsubstituiertes oder durch Niederalkyl substituiertes Pyridyl, welches am Stickstoff unsubstituiert oder durch Sauerstoff substituiert ist, bedeutet, $R_2$ und $R_3$ unabhängig voneinander je Wasserstoff oder Niederalkyl bedeuten, einer oder zwei der Reste $R_4$, $R_5$, $R_6$, $R_7$ und $R_8$ je Nitro, fluorsubstituiertes Niederalkoxy oder einen Rest der Formel II bedeuten, worin $R_9$ für Wasserstoff oder Niederalkyl, X für Oxo, Thio, Imino, N-Niederalkyl-imino, Hydroximino oder O-Niederalkyl-hydroximino, Y für Sauerstoff oder die Gruppe NH, n für 0 oder 1 und $R_{10}$ für einen aliphatischen Kohlenwasserstoffrest mit 5-22 C-Atomen, für einen Phenyl- oder Naphthylrest, welcher jeweils unsubstituiert oder durch Cyano, Niederalkyl, Hydroxy-niederalkyl, Aminoniederalkyl, (4-Methyl-piperazinyl)-niederalkyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder durch Niederalkoxycarbonyl substituiert ist, für Phenylniederalkyl, worin der Phenylrest unsubstituiert oder wie vorstehend angegeben

substituiert ist, für einen Cycloalkyloder Cycloalkenylrest mit bis zu 30 C-Atomen, für Cycloalkyl-niederalkyl oder Cycloalkenyl-niederalkyl mit jeweils bis zu 30 C-Atomen im Cycloalkyl- oder Cycloalkenylteil, für einen monocyclischen Rest mit 5 oder 6 Ringgliedern und 1-3 Ringatomen, ausgewählt aus Stickstoff, Sauerstoff und Schwefel, an welchen ein oder zwei Benzolreste annelliert sein können, oder für Niederalkyl, welches durch einen solchen monocyclischen Rest substituiert ist, steht, und die übrigen der Reste $R_4$, $R_5$, $R_6$, $R_7$ und $R_8$ unabhängig voneinander je Wasserstoff, Niederalkyl, welches unsubstituiert oder durch Amino Niederalkylamino, Diniederalkylamino, Piperazinyl, Piperidinyl, Pyrrolidinyl oder durch Morpholinyl substituiert ist, Niederalkanoyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder Niederalkoxycarbonyl bedeuten, und Salze von solchen Verbindungen mit mindestens einer salzbildenden Gruppe.

Besonders bevorzugt sind Verbindungen der Formel I, worin $R_1$ an einem Ringkohlenstoffatom gebundenes Pyridyl, welches am Stickstoff unsubstituiert oder durch Sauerstoff substituiert ist, bedeutet, $R_2$ und $R_3$ je Wasserstoff bedeuten, $R_4$ Wasserstoff oder Niederalkyl bedeutet, $R_5$ Wasserstoff, Niederalkyl oder fluorsubstituiertes Niederalkoxy bedeutet, $R_6$ Wasserstoff bedeutet, $R_7$ Nitro, fluorsubstituiertes Niederalkoxy oder einen Rest der Formel II bedeutet, worin $R_9$ für Wasserstoff, X für Oxo, n für 0 und $R_{10}$ für einen aliphatischen Kohlenwasserstoffrest mit 5-22 C-Atomen, für einen Phenylrest, welcher unsubstituiert oder durch Cyano, Niederalkyl, (4-Methyl-piperazinyl)-niederalkyl, Niederalkoxy, Halogen oder durch Carboxy substituiert ist, für einen Cycloalkylrest mit bis zu 30 C-Atomen oder für einen monocyclischen Rest mit 5 oder 6 Ringgliedern und 1-3 Schwefelringatomen stehen, und $R_8$ Wasserstoff bedeutet, und pharmazeutisch verwendbare Salze von solchen Verbindungen mit mindestens einer salzbildenden Gruppe.

Besonders bevorzugt sind insbesondere Verbindungen der Formel I, worin $R_1$ jeweils an einem Kohlenstoffatom gebundenes Pyridyl oder N-Oxido-pyridyl bedeutet, $R_2$ und $R_3$ je Wasserstoff bedeuten, $R_4$ Wasserstoff oder Niederalkyl bedeutet, $R_5$ Wasserstoff, Niederalkyl oder Trifluormethyl bedeutet, $R_6$ Wasserstoff bedeutet, $R_7$ für Nitro, fluorsubstituiertes Niederalkoxy oder einen Rest der Formel II steht, worin $R_9$ Wasserstoff, X Oxo, n die Zahl 0 und $R_{10}$ an einem Kohlenstoffatom gebundenes Pyridyl, unsubstituiertes oder durch Halogen, Cyano, Niederalkoxy, Carboxy, Nierderalkyl oder 4-Methyl-piperazinyl-methyl substituiertes Phenyl, $C_5$-$C_7$-Alkyl, Thienyl, 2-Naphthyl oder Cyclohexyl bedeuten, und $R_8$ Wasserstoff bedeutet, und pharmazeutisch verwendbare Salze von solchen Verbindungen mit mindestens einer salzbildenden Gruppe.

In erster Linie bevorzugt sind insbesondere Verbindungen der Formel I, worin $R_4$ und $R_8$ je für Wasserstoff stehen oder worin mindestens einer der Reste $R_4$ und $R_8$ für Niederalkyl steht, und der andere der Reste $R_4$ und $R_8$ und die übrigen Reste die obengenannten Bedeutungen haben, und pharmazeutisch verwendbare Salze von solchen Verbindungen mit mindestens einer salzbildenden Gruppe.

In erster Linie bevorzugt sind insbesondere Verbindungen der Formel I, worin $R_1$ an einem Kohlenstoffatom gebundenes Pyridyl bedeutet, $R_2$, $R_3$, $R_4$, $R_5$, $R_6$ und $R_8$ je Wasserstoff bedeuten und $R_7$ für Nitro oder einen Rest der Formel II steht, worin $R_9$ Wasserstoff, X Oxo, n die Zahl 0 und $R_{10}$ an einem Kohlenstoffatom gebundenes Pyridyl, unsubstituiertes oder durch Fluor, Chlor, Cyano, Niederalkoxy, Carboxy, Niederalkyl oder 4-Methyl-piperazinyl-methyl substituiertes Phenyl, $C_5$-$C_7$-Alkyl, Thienyl oder Cyclohexyl bedeuten, und pharmazeutisch verwendbare Salze davon.

Am meisten bevorzugt sind die in den Beispielen beschriebenen Verbindungen der Formel I und pharmazeutisch verwendbare Salze von solchen Verbindungen mit mindestens einer salzbildenden Gruppe.

Im Hinblick auf die Hemmung der Proteinkinase C sind diejenigen obengenannten Verbindungen der Formel I am meisten bevorzugt, worin $R_4$ und $R_8$ je für Wasserstoff stehen und die übrigen Substituenten die obengenannten Bedeutungen haben, und pharmazeutisch verwendbare Salze von solchen Verbindungen mit mindestens einer salzbildenden Gruppe.

Die Verbindungen der Formel I und Salze solcher Verbindungen mit mindestens einer salzbildenden Gruppe werden nach an sich bekannten Verfahren hergestellt. Das erfindungsgemässe Verfahren ist dadurch gekennzeichnet, dass man

a) eine Verbindung der Formel III,

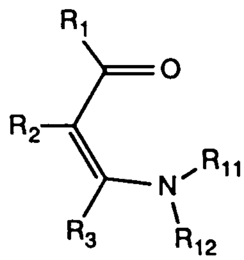

(III)

worin $R_{11}$ und $R_{12}$ unabhängig voneinander je für Niederalkyl stehen und $R_1$, $R_2$ und $R_3$ die obengenannten Bedeutungen haben, wobie in einer Verbindung der Formel III vorhandene funktionelle Gruppen mit Ausnahme der an der Reaktion teilnehmenden Gruppen erforderlichenfalls in geschützter From vorliegen, oder ein Salz einer solchen Verbindung mit einer Verbindung der Formel IV,

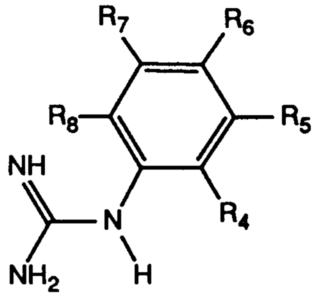

(IV)

worin die Substituenten die obengenannten Bedeutungen haben, wobei in einer Verbindung der Formel IV vorhandene funktionelle Gruppen mit Ausnahme der an der Reaktion teilnehmenden Guanidinogruppe erforderlichenfalls in geschützter From vorliegen, oder mit einem Salz einer solchen Verbindung umsetzt, und vorhandene Schutzgruppen abspaltet, oder

b) zur Herstellung einer Verbindung der Formel I, worin die Reste $R_4$, $R_5$, $R_6$, $R_7$ und $R_8$ die obengenannten Bedeutungen mit Ausnahme von Nitro und fluorsubstituiertem Niederalkoxy haben, eine Verbindung der Formel V,

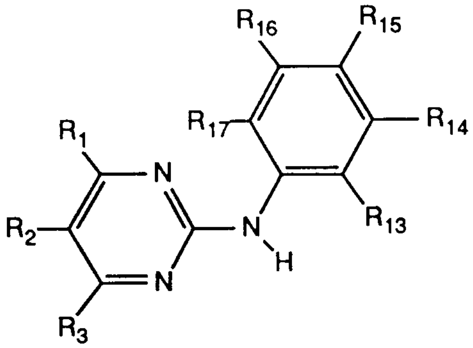

(V)

worin einer oder zwei der Reste $R_{13}$, $R_{14}$, $R_{15}$, $R_{16}$ und $R_{17}$ je Amino und die übrigen der Reste $R_{13}$, $R_{14}$, $R_{15}$, $R_{16}$ und $R_{17}$ unabhängig voneinander je Wasserstoff, Niederalkyl, welches unsubstituiert oder durch freies oder alkyliertes Amino, Piperazinyl, Piperidinyl, Pyrrolidinyl oder Morpholinyl substituiert ist, Niederalkanoyl, Trifluormethyl, freies, verethertes oder verestertes Hydroxy, freies, alkyliertes oder acyliertes Amino oder freies oder verestertes Carboxy bedeuten, und die übrigen Substituenten die obengenannten Bedeutungen haben, wobei in einer Verbindung der Formel V vorhandene funktionelle Gruppen mit Ausnahme der an der Reaktion teilnehmenden Aminogruppe(n) erforderlichenfalls in geschützter Form vorliegen, mit einer Verbindung der Formel VI,

$$HO\text{-}C(=X)\text{-}(Y)_n\text{-}R_{10} \qquad (VI)$$

worin die Substituenten und Symbole die obengenannten Bedeutungen haben, wobei in einer Verbindung der Formel VI vorhandene funktionelle Gruppen mit Ausnahme der an der Reaktion teilnehmenden HO-C(=X)-Gruppe erforderlichenfalls in geschützter Form vorliegen, oder mit einem reaktionsfähigen Derivat einer Verbindung der Formel VI umsetzt und vorhandene Schutzgruppen abspaltet, oder

c) zur Herstellung einer Verbindung der Formel I, worin $R_1$ Pyridyl bedeutet, welches am Stickstoff durch Sauerstoff substituiert ist, und worin die übrigen Substituenten und Symbole die obengenannten Bedeutungen haben, eine Verbindung der Formel I, worin $R_1$ Pyridyl bedeutet, mit einem geeigneten Oxidationsmittel in die N-Oxido-Verbindung überführt,

und wenn erwünscht, eine nach einem der Verfahren a bis c erhaltene Verbindung der Formel I in ihr Salz überführt, oder ein erhaltenes Salz einer Verbindung der Formel I in die freie Verbindung umwandelt.

Die Durchführung der obengenannten Verfahrensvarianten wird im folgenden näher erläutert:

Allgemeines:

Die Endstoffe der Formel I können Substituenten enthalten, die auch als Schutzgruppen in Ausgangsstoffen zur Herstellung anderer Endstoffe der Formel I verwendet werden können. Als "Schutzgruppe" wird daher im Rahmen dieses Textes, wenn aus dem Zusammenhang nicht anders ersichtlich, nur eine solche leicht abspaltbare Gruppe bezeichnet, die nicht Bestandteil des jeweils gewünschten Endstoffes der Formel I ist.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thiaaliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Aminogruppe kann z.B. in Form einer leicht spaltbaren Acylamino-, Arylmethylamino-, verätherten Mercaptoamino-, 2-Acyl-nieder-alk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicyclohexylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B.

Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl-und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert-Butyl, Niederalkoxy, wie Methoxy, Halogen, wie Chlor, und/oder Nitro substituiertes Phenyl ist Eine entsprechende Aminoschutzgruppe ist z.B. 4-Nitrophenylthio.

In einem als Aminoschutzgruppe verwendbaren 2-Acyl-niederalk-1-en-1-ylrest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niedealkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogenniederalkoxycarbonyl, wie 2,2,2-Trichlorethoxycarbonyl, ferner Trityl oder Formyl. Die Abspaltung der Schutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, gespalten werden, und eine mit einer organischen Silylgruppe geschützte Aminogruppe kann z.B. mittels Hydrolyse oder Alkoholyse freigesetzt werden. Eine durch 2-Halogenacetyl, z.B. 2-Chloracetyl, geschützte Aminogruppe kann durch Behandeln mit Wasserstoff in Gegenwart einer Base, oder mit einem Thiolatsalz, wie einem Alkalimettalthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwasserstoffsäure in die freie Aminogruppe übergeführt werden.

Eine durch eine geeignete Acylgruppe, eine organische Silylgruppe oder durch gegebenenfalls substituiertes 1-Phenylniederalkyl geschützte Hydroxygruppe kann analog einer entsprechend geschützten Aminogruppe freigesetzt. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators, freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt. Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

Verfahren a:

Vorzugsweise stehen $R_{11}$ und $R_{12}$ je für Methyl.

Freie funktionelle Gruppen in einer Verbindung der Formel III, die vorteilhafterweise durch leicht abspaltbare Schutzgruppen geschützt sind, sind insbesondere Aminogruppen im Rest $R_1$ sowie die Iminogruppe von 1H-Indolyl. Letztere kann z.B. durch Benzyl geschützt sein.

Freie funktionelle Gruppen in einer Verbindung der Formel IV, die vorteilhafterweise durch leicht abspaltbare Schutzgruppen geschützt sind, sind insbesondere Amino-, aber auch Hydroxy- und Carboxygruppen.

Ein Salz einer Verbindung der Formel IV ist vorzugsweise ein Säureadditionssalz, z.B. ein Nitrat oder eines der für die Endstoffe der Formel I genannten Säureadditionssalze.

Die Reaktion wird in einem geeigneten Lösungs- oder Suspensionsmittel, z.B. einem geeigneten Alkohol, wie 2-Methoxy-ethanol oder einem geeigneten Niederalkanol, z.B. Isopropanol, bei einer Temperatur zwischen Raumtemperatur (ca. 20 °C) und 150 °C, z.B. unter Rückfluss, durchgeführt. Insbesondere wenn die Verbindung der Formel IV als Salz eingesetzt wird, wird dieses Salz, vorzugsweise in situ, durch Zugabe einer geeigneten Base, wie einem Alkalimetallhydroxid, z.B. Natriumhydroxid, in die freie Verbindung überführt.

Vorzugsweise geht man von Verbindungen der Formel IV aus, worin einer oder zwei der Reste $R_4$, $R_5$, $R_6$, $R_7$ und $R_8$ je Nitro und die übrigen der Reste $R_4$, $R_5$, $R_6$, $R_7$ und $R_8$ unabhängig voneinander je Wasserstoff, Niederalkyl, welches unsubstituiert oder durch freies oder alkyliertes Amino, Piperazinyl, Piperidinyl, Pyrrolidinyl oder Morpholinyl substituiert ist, Niederalkanoyl, Trifluormethyl, freies, verethertes oder verestertes Hydroxy, freies, alkyliertes oder acyliertes Amino oder freies oder verestertes Carboxy bedeuten.

Das Ausgangsmaterial der Formel III erhält man durch Umsetzung einer Verbindung der Formel VII,

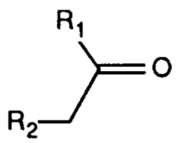

(VII)

worin die Substituenten die obengenannten Bedeutungen haben, mit einer Verbindung der Formel VIII,

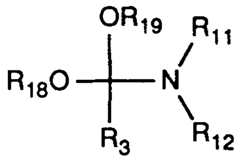

(VIII)

worin $R_{18}$ und $R_{19}$ je für Niederalkyl stehen und die übrigen Substituenten die obengenannten Bedeutungen haben, analog wie beschrieben in der Europäischen Patentanmeldung mit der Veröffentlichungsnummer 233461. Typische Vertreter einer Verbindung der Formel VIII sind N,N-Dimethyl-formamid-dimethylacetal und N,N-Dimethyl-acetamiddimethylacetal. Die Reaktion erfolgt bei mehrstündigem, z.B. 4-24stündigem Erwärmen der Reaktanden der Formeln VII und VIII, in Abwesenheit oder, falls erforderlich, Anwesenheit eines Lösungsmittels auf eine Temperatur zwischen etwa 50 °C und 150 °C.

Das Ausgangsmaterial der Formel III erhält man alternativ auch durch Umsetzung einer Verbindung der Formel VII mit einem Ester der Formel $R_3$-C(=O)-O-$CH_2$-$CH_3$, worin $R_3$ die obengenannte Bedeutung hat, und Reaktion des erhaltenen Produkts mit einem Amin der Formel H-N($R_{11}$)-$R_{12}$, worin die Substituenten die obengenannten Bedeutungen haben.

Das Ausgangsmaterial der Formel IV erhält man in Form eines Säureadditionssalzes durch Umsetzung einer Verbindung der Formel IX,

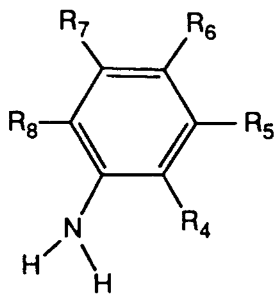

(IX)

worin die Substituenten die obengenannten Bedeutungen haben, mit Cyanamid ($NC-NH_2$). Die Reaktion erfolgt in einem geeigneten Lösungs- oder Suspensionsmittel, z.B. einem geeigneten Alkohol, z. B. einem geeigneten Niederalkanol, wie Ethanol, in Gegenwart äquimolarer Mengen der salzbildenden Säure bei einer Temperatur zwischen Raumtemperatur und 150 °C, z.B. unter Rückfluss.

Verfahren b:

Freie funktionelle Gruppen in einer Verbindung der Formel V oder VI, die vorteilhafterweise durch leicht abspaltbare Schutzgruppen geschützt sind, sind insbesondere Amino-, aber auch Hydroxy- und Carboxygruppen, die an der gewünschten Reaktion nicht teilnehmen sollen, z.B. Amino im Rest $R_1$.

Ein reaktionsfähiges Derivat einer Verbindung der Formel VI, worin X für Oxo steht, ist insbesondere ein reaktionsfähiger (aktivierter) Ester, ein reaktionsfähiges Anhydrid oder ein reaktionsfähiges cyclisches Amid. Analoges gilt für die Derivate, worin X eine der übrigen obengenannten Bedeutungen hat.

Reaktionsfähige (aktivierte) Ester einer Säure der Formel VI sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; CyanamidMethode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenylthioester (die man z.B. durch Behandeln der entsprechenden Säure mit gegebenenfalls, z. B. durch Nitro, substituierten Thiophenolen, u.a. mit Hilfe der Anhydrid- oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride einer Säure der Formel VI können symmetrische oder vorzugsweise gemischte Anhydride dieser Säure sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechlorimethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan - oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimettallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid--Methode), oder Pyrazolen, z.B. 3,5-Dimethyl-pyrazol (die man z.B. über das

Säurehydrazid durch Behandeln mit Acetylaceton erhalten kann; Pyrazolid-Methode).

Derivate von Säuren der Formel VI, die als Acylierungsmittel verwendet werden, können auch in situ gebildet werden. So kann man z.B. N,N'-di-substituierte Amidinoester in situ bilden, indem man das Gemisch des Ausgangsmaterials der Formel V und der als Acylierungsmittel verwendeten Säure in Gegenwart eines geeigneten N,N-disubstituierten Carbodiimids, z.B. N,N'-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel V bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Vorzugsweise wird die Reaktion so durchgeführt, dass man ein reaktionsfähiges Carbonsäurederivat einer Verbindung der Formel VI mit einer Verbindung der Formel V umsetzt, worin die an der Reaktion teilnehmende Amino- oder Hydroxygruppe in freier Form vorliegt.

Die Reaktion kann in an sich bekannter Weise durchgeführt werden, wobei die Reaktionsbedingungen in erster Linie davon abhängen, ob und wie die Carboxylgruppe des Acylierungsmittels aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes mittel sein kann, unter Kühlen oder Erwärmen, z.B. in einem Temperaturbereich von etwa -30°C bis etwa +150°C, insbesondere von etwa 0°C bis + 100°C, vorzugsweise von Raumtemperatur (ca. +20°C) bis +70°C, in einem offenen oder geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Dicyclohexyl-oder N-Ethyl-N'(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Ka!iumcarbonat oder -hydrogencarbonat (üblicherweise zusammen mit einem Sulfat), oder organische Basen, wie üblicherweise Pyridin oder sterisch gehinderte Triniederalkylamine, z.B. N,N-Diisopropyl-N-ethyl-amin.

Das Ausgangsmaterial der Formel V erhält man durch Reduktion der Nitrogruppe(n) in einer Verbindung der Formel I, worin einer oder zwei der Reste $R_4$, $R_5$, $R_6$, $R_7$ und $R_8$ je Nitro bedeuten. Diese Reduktion kann z.B. durch katalytische Hydrierung in einem geeigneten Lösungsmittel, wie einem geeigneten acyclischen oder cyclischen Ether, wie in Tetrahydrofuran, durchgeführt werden. Als Hydrierkatalysator verwendet man vorzugsweise Palladium auf Aktivkohle (5 %) und führt in diesem Fall die Hydrierung vorzugsweise unter Normaldruck durch.

Verfahren c:

Ein geeignetes Oxidationsmittel zur Überführung einer Verbindung der Formel I, worin $R_1$ Pyridyl bedeutet, in die N-Oxido-Verbindung ist vorzugsweise eine geeignete Persäure, z.B. eine geeignete Perbenzoesäure, wie insbesondere m-Chlor-perbenzoesäure. Die Reaktion wird in einem inerten Lösungsmittel, z.B. einem halogenierten Kohlenwasserstoff, wie vorzugsweise Methylenchlorid, bei Temperaturen zwischen etwa -20°C und +150 °C, hauptsächlich zwischen etwa 0°C und dem Siedepunkt des betreffenden Lösungsmittels, im allgemeinen unterhalb von +100°C, und vorzugsweise bei Raumtemperatur oder leicht erhöhter Temperatur (20°C-70°C) durchgeführt.

Säureadditionssalze von Verbindungen der Formel I erhält man in üblicher Weise, z.B. durch Behandeln mit einer Säure oder einem geeigneten Anionenaustauschreagens.

Säureadditionssalze können in üblicher Weise in die freien Verbindungen überführt werden, z.B. durch Behandeln mit einem geeigneten basischen Mittel.

Gemische von Isomeren können in an sich bekannter Weine, z.B. durch fraktonierte Kristallisation, Chromatographie etc. in die einzelnen Isomeren aufgetrennt werden.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in An- oder Abwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, insbesondere von etwa 0°C bis etwa +70°C, vorzugsweise von etwa +10°C bis etwa +50°C, hauptsächlich bei Raumtemperatur, in einem geeigneten Gefäss und erforderlichenfalls in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betrifft auch ein Verfahren zur Behandlung von Warmblütern, die an einer Tumorerkrankung leiden, wobei man Warmblütern, die einer solchen Behandlung bedürfen, eine wirksame tumorhemmende Menge einer Verbindung der Formel I oder eines pharmazeutisch verwendbaren Salzes davon verabreicht. Die Erfindung betrifft ausserdem die Verwendung einer Verbindung der Formel I oder eines pharmazeutisch verwendbaren Salzes davon zur Hemmung der PDGF-Rezeptor-Kinase oder die Verwendung einer Verbindung der Formel I, worin $R_4$ und $R_8$ je Wasserstoff bedeuten, oder eines pharmazeutisch verwendbaren Salzes davon zur Hemmung der Proteinkinase C bei Warmblütern oder zur Herstellung von pharmazeutischen Präparaten zur Anwendung zur therapeutischen Behandlung des menschlichen oder tierischen Körpers. Dabei werden an einen Warmblüter von etwa 70 kg Körpergewicht je nach Spezies, Alter, individuellem Zustand, Applikationsweise und dem jeweiligen Krankheitsbild wirksame Dosen, z.B. tägliche Dosen von etwa 1-1000 mg, insbesondere 50-500 mg verabreicht.

Die Erfindung betrifft auch pharmazeutische Präparate, die eine wirksame Menge, insbesondere eine zur Prophylaxe oder Therapie einer der obengenannten Krankheiten wirksame Menge, der Aktivsubstanz zusammen mit pharmazeutisch verwendbaren Trägerstoffen enthalten, die sich zur topischen, enteralen, z.B. oralen oder rektalen, oder parenteralen Verabreichung eignen, und anorganisch oder organisch, fest oder flüssig sein können. Zur oralen Verabreichung verwendet man insbesondere Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmacksstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 1 % bis 100 %, insbesondere von etwa 1 % bis etwa 20 %, des bzw. der Aktivstoffe.

Die nachfolgenden Beispiele illustrieren die Erfindung, ohne sie in irgendeiner Form einzuschränken. Die $R_f$-Werte werden auf Kieselgeldünnschichplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben.

<u>Abkürzungen:</u>

HV: Hochvakuum
RT: Raumtemperatur
n: normal (geradkettig)

<u>Beispiel 1:</u> Zu einer Lösung von 30 g (0,17 Mol) 3-Dimethylamino-1-(3-pyridyl)-2-propen-1-on [beschrieben in EP-A-0 233 461] in 250 ml Isopropanol werden 41,3 g (0,17 Mol) 3-Nitro-phenyl-guanidin-nitrat, aufgeschlämmt in 50 ml Isopropanol, zugegeben. Nach der Zugabe von 7,49 g (0,19 Mol) Natriumhydroxid wird die gelbe Suspension 8 Stunden am Rückfluss gekocht. Nach dem Abkühlen auf 0° wird abfiltriert und mit 200 ml Isopropanol nachgewaschen.

Das Nutschgut wird in 300 ml Wasser aufgeschlämmt und 30 Min. rühren gelassen, filtriert und mit 200 ml Wasser gewaschen. Nach nochmaligem Aufschlämmen in 200 ml Ethanol und Waschen mit 200 ml Ethanol-Diethylether (1:1) erhält man N-(3-Nitro-phenyl)-4-(3-pyridyl)-2-pyrimidinamin; Smp. 212-213°, $R_f$ = 0,75 (Chloroform:Methanol = 9:1).

Das Ausgangsmaterial erhält man folgendermassen:

Stufe 1.1: Zu einer gelben Suspension von 82,88g (0,6 Mol) 3-Nitro-anilin in 200 ml Ethanol werden 42 ml (0,6 Mol) Salpetersäure (65%ig) zugetropft. Nachdem die exotherme Reaktion abgeklungen ist, gibt man 75,7 g (0,9 Mol) Cyanamid (50% in Wasser) zu und kocht die Reaktionsmischung 21 Stunden am Rückfluss. Nach dem Abkühlen auf 0° wird abfiltriert und sechsmal mit Ethanol-Diethylether (1:1) nachgewaschen. Nach dem Trocknen am HV bei 40° erhält man 3-Nitro-phenyl-guanidin-nitrat; Smp. 205-207°.

Stufe 1.2: 8 g (0,35 Mol) Natrium werden in 260 ml Toluol vorgelegt und bei 100° mit einem Vibromischer suspendiert. Nach dem Abkühlen auf 0° werden unter Kühlung 17 ml (0,42 mol) Methanol zugetropft und anschliessend wird 45 Min. bei 75° gerührt. Bei 25° wird unter Eiskühlung eine Lösung aus 38,5 ml (0,35 Mol) 3-Acetyl-pyridin, und 28 ml (0,35 Mol) Ameisensäureethylester in 300 ml Toluol innerhalb von 45 Min. zugetropft. Die gelbe Suspension wird 16 Std. bei 25° gerührt und anschliessend mit 23,7 g (0,52 Mol) Dimethylamin versetzt. Nach der Zugabe von 100 ml Toluol wird 45 Min. bei 25° gerührt, dann bei 0° eine Lösung aus 20 ml Essigsäure in 150 ml Toluol innerhalb von 30 Min. zugetropft und anschliessend 1 Std. am Rückfluss gekocht. Nach dem Abkühlen auf 25° wird abfiltriert, mit 500 ml Toluol-Hexan (1:1) gewaschen und das Filtrat bis zum Beginn der Kristallisation eingeengt. Abkühlen auf 0°, Abfiltrieren und Trocknen bei 80 ° im HV ergibt 3-Dimethylamino-1-(3-pyridyl)-2-propen-1-on; Smp. 81-82°.

Beispiel 2: 100 mg (0,38 mMol) N-(3-Amino-phenyl)-4-(3-pyridyl)-2-pyrimidinamin werden in 5ml Pyridin gelöst, mit 58,5 µl (0,46mmol) 4-Chlor-benzoylchlorid versetzt und 24 Stunden bei Raumtemperatur gerührt. Das Reaktionsgemisch wird mit 10 ml Wasser versetzt, auf O° gekühlt und abfiltriert. Nach dem Waschen mit Wasser und Trocknen erhält man N-[3-(4-Chlorbenzoylamido)-phenyl]-4-(3-pyridyl)-2-pyrimidinamin; Smp. 238-240°, $R_f$ = 0,66 (Chloroform:Methanol = 9:1).

Das Ausgangsmaterial erhält man folgendermassen:

Stufe 2.1: Eine Suspension von 17,0 g (0,058 Mol) N-(3-Nitro-phenyl)-4-(3-pyridyl)-2-pyrimidinamin in 1700 ml Tetrahydrofuran wird mit 1,7 g Palladium auf Aktivkohle (5 %) unter einer Wasserstoffatmosphäre bei Normaldruck während 21 Stunden gerührt. Die Suspension wird filtriert und das Filtrat am Rotationsverdampfer eingeengt. Das zurückgebliebene gelbe Festprodukt wird über Nacht in 200 ml Methylenchlorid geführt. Nach der Filtration und dem Trocknen erhält man N-(3-Amino-phenyl)-4-(3-pyridyl)-2-pyrimidinamin; Smp. 89-90°, $R_f$ = 0,38 (Chloroform:Methanol = 9:1).

Beispiel 3: Eine Lösung von 100 mg (0,38 mMol) N-(3-Amino-phenyl)-4-(3-pyridyl)-2-pyrimidinamin in 5 ml Pyridin wird mit 53 µl (0,46 mMol) Benzoylchlorid versetzt und unter einer Stickstoffatmosphäre während 24 Stunden bei Raumtemperatur gerührt. Die Reaktionsmischung wird mit 10 ml Wasser versetzt, auf 0° abgekühlt, abfiltriert und mit Wasser nachgewaschen. Nach dem Trocknen am HV erhält man N-(3-Benzoylamidophenyl)-4-(3-pyridyl)-2-pyrimidinamin; Smp. 207-209°, $R_f$ = 0,53 (Chloroform:Methanol = 9:1).

Beispiel 4: Eine Lösung von 100 mg (0,38 mMol) N-(3-Amino-phenyl)-4-(3-pyridyl)-2-pyrimidinamin und 59 mg (0,46 mMol) 2-Pyridincarbonsäurechlorid in 5 ml pyridin wird bei RT unter Stickstoff während 24 Std. gerührt. Nach der Zugabe von 30 mg (0,23 mMol) 2-Pyridincarbonsäurechlorid wird 18 Stunden gerührt, dann gibt man weitere 25mg (0,19 mMol) 2-Pyridincarbonsäurechlorid zu und lässt 72 Std. bei 25° rühren. Nach der Zugabe von 10 ml Wasser und Abkühlen auf 0° wird abfiltriert und mit Wasser gewaschen. Eine chromatographische Trennung (Kieselgel, $CHCl_3$/MeOH = 9:1) ergibt N-[3-(2-Pyridyl)-carboxamido-phenyl]-4-(3-pyridyl)-2-pyrimidinamin; Smp. 187-190°, $R_f$ = 0,58 (Chloroform:Methanol = 9:1).

Beispiel 5: Analog Beispiel 4 wird aus 3-Pyridincarbonsäurechlorid das N-[3-(3-Pyridyl)-carboxamido-phenyl]-4-(3-pyridyl)-2-pyrimidinamin hergestellt; Smp. 217-220°, $R_f$ = 0,29 (Chloroform:Methanol = 9:1).

Beispiel 6: Analog Beispiel 4 wird aus 4-Pyridincarbonsäurechlorid das N-[3-(4-Pyridyl)-carboxamido-phenyl]-4-(3-pyridyl)-2-pyrimidinamin synthetisiert; Smp. 224-226°, $R_f$ = 0,29 (Chloroform:Methanol = 9:1).

Beispiel 7: Eine Lösung von 100 mg (0,38 mMol) N-(3-Amino-phenyl)-4-(3-pyridyl)-2-pyrimidinamin in 5 ml Pyridin wird mit 63 µl (0,46 mMol) Pentafluor-benzoylchlorid versetzt und unter Stickstoff bei RT während 17 Std. gerührt. Die braune Reaktionslösung wird mit 10 ml Wasser versetzt, auf 0° abgekühlt und abfiltriert. Der Rückstand wird aus Ethanol-Aceton umkristallisiert und ergibt das kristalline Produkt N-(3-Pentafluorbenzoylamido-phenyl)-4-(3-pyridyl)-2-pyrimidinamin; Smp. 234-244°, $R_f$ = 0,41 (Chloroform:Methanol 9:1).

Beispiel 8: 28 mg (0,19 mMol) Phthalsäureanhydrid werden zu einer Lösung von 50 mg (0,19 mMol) N-(3-Amino-phenyl)-4-(3-pyridyl)-2-pyrimidinamin 1 ml Pyridin gegeben. Nach 2,5 Stunden wird die gelbe Reaktionslösung mit weiteren 14 mg (0,095 mMol) Phthalsäureanhydrid versetzt und während 20 Std. bei 25° gerührt. Die Suspension wird filtriert, mit wenig kaltem Pyridin gewaschen. Der Rückstand wird zweimal mit je 2,5 ml absolutem Ethanol digeriert und ergibt N-[3-(2-carboxy-benzoylamido)-phenyl]-4-(3-pyridyl)-2-pyrimidinamin; Smp. 206-209°, $R_F$ = 0,07 (Chloroform:Methanol = 9:1).

Beispiel 9: Eine Lösung von 100 mg (0,38 mMol) N-(3-Amino-phenyl)-4-(3-pyridinyl)-2-pyrimidinamin und 105 µl (0,46 mMol) Capronsäureanhydrid in 5 ml Pyridin wird 24 Std. bei 25° unter einer Stickstoffatmosphäre gerührt und

dann am Rotationsverdampfer eingeengt. Der Rückstand wird mit einer Flash-Chromatographie (Kieselgel, Chloroform:Methanol 95:5) gereinigt, worauf man N-(3-n-Hexanoylamido-phenyl)-4-(3-pyridyl)-2-pyrimidinamin erhält; Smp. 180-184°, $R_f$ = 0,78 (Chloroform:Methanol = 9:1).

Beispiel 10: 1 g (5,68 mMol) 3-Dimethylamino-1-(2-pyridyl)-2-propen-1-on [EP-A-233461] wird in 8 ml Isopropanol gelöst und mit 1,38 g (5,68 mMol) 3-Nitrophenylguanidin-Nitrat versetzt. Nach der Zugabe von 0,25g (6,24 mMol) Natriumhydroxid wird die gelbe Suspension 20 Std. am Rückfluss erhitzt, anschliessend auf 0° abgekühlt, abfiltriert und mit 30 ml Isopropanol nachgewaschen. Das Nutschgut wird 20 Min. in 15 ml Ethanol gerührt, abfiltriert und mit wenig kaltem Ethanol gewaschen. Man erhält N-(3-Nitro-phenyl)-4-(2-pyridyl)-2-pyrimidinamin; Smp. 213-219°.

Beispiel 11: Zu einer Lösung von 1 g (5,68 mMol) 3-Dimethylamino-1-(4-pyridyl)-2-propen-1-on [U.S. Patent 4281000] in 8 ml Isopropanol werden 1,38 g (5,68 mMol) 3-Nitro-phenylguanidin-Nitrat und 0,25 g (6,24 mMol) Natriumhydroxid zugegeben. Die gelbe Suspension wird 20 Std. am Rückfluss erhitzt und anschliessend auf 0° abgekühlt. Nach dem Waschen mit 30 ml Isopropanol wird das Nutschgut nacheinander in 15 ml Ethanol und dann in 15 ml Wasser aufgeschlämmt und jeweils abfiltriert. Nach dem Trocknen am HV erhält man N-(3-Nitro-phenyl)-4-(4-pyridyl)-2-pyrimidinamin; Smp. 282-284°.

Beispiel 12: Analog Beispiel 2 wird aus 2-Methoxy-benzoylchlorid das N-[3-(2-Methoxy-benzoylamido)-phenyl]-4-(3-pyridyl)2-pyrimidinamin hergestellt; Smp. 115-117°, $R_f$ = 0,76 (Chloroform:Methanol = 9:1).

Beispiel 13: Analog Beispiel 2 wird aus 4-Fluor-benzoylchlorid das N-[3-(4-Fluorbenzoylamido)-phenyl]-4-(3-pyridyl)-2-pyrimidinamin hergestellt; Smp.: 215-216°, $R_f$ = 0,34 (Chloroform:Methanol = 9:1).

Beispiel 14: Analog Beispiel 2 wird aus 4-Cyano-benzoylchlorid das N-[3-(4-Cyanobenzoylamido)-phenyl]-4-(3-pyridyl)-2-pyridinamin hergestellt; Smp. 220-222°, $R_f$ = 0,31 (Chloroform:Methanol = 9:1).

Beispiel 15: Analog Beispiel 2 wird aus 2-Thiophencarbonsäurechlorid das N-[3-(2-Thienylcarboxamido)-phenyl]-4-(3-pyridyl)-2-pyrimidinamin hergestellt; Smp. 139-141°; $R_f$ = 0,35 (Chloroform:Methanol = 9:1).

Beispiel 16: Analog Beispiel 2 wird aus Cyclohexancarbonsäurechlorid das N-(3-Cyclohexylcarboxamido-phenyl)-4-(3-pyridyl)-2-pyrimidinamin hergestellt; Smp. 205-206°, $R_f$ = 0,36 (Chloroform:Methanol = 9:1).

Beispiel 17: Analog Beispiel 2 wird aus 4-Methyl-benzoylchlorid das N-[3-(4-Methylbenzoylamido)-phenyl]-4-(3-pyridyl)-2-pyrimidinamin hergestellt; Smp. 214-216°, $R_f$ = 0,64 (Chloroform:Methanol = 9:1).

Beispiel 18: Analog Beispiel 2 wird bei der Behandlung von 100 mg (0,38 mMol) N-(3-Amino-phenyl)-4-(4-pyridyl)-2-pyrimidinamin mit 58 µl (0,46 mMol) 4-Chlor-benzoylchlorid das N-[3-(4-Chlor-benzoylamido)-phenyl]-4-(4-pyridyl)-2-pyrimidinamin hergestellt; Smp. 258-261°, $R_f$ = 0,37 ($CHCl_3$:Methanol = 9:1).

Das Ausgangsmaterial erhält man folgendermassen:

Stufe 18.1: Analog Stufe 2.1 erhält man bei der Behandlung von 300 mg (1,0 mMol) N-(3-Nitro-phenyl)-4-(4-pyridyl)-2-pyrimidinamin (siehe Beispiel 11) unter einer Wasserstoffatmosphäre N-(3-Amino-phenyl)-4-(4-pyridyl)-2-pyrimidinamin; Smp. 200-202°, $R_f$ = 0,27 ($CHCl_3$:Methanol = 95:5).

Beispiel 19: Analog Beispiel 2 wird aus 98 mg (0,3 mMol) 4-(4-Methyl-piperazinomethyl)-benzoylchlorid N-{3-[4-(4-Methyl-piperazinomethyl)-benzoylamido]-phenyl}-4-(3-pyridyl)-2-pyrimidinamin hergestellt; Smp. 198-201°.

Beispiel 20: Eine Lösung von 8,0 g (28,85 mMol) N-(5-Amino-2-methyl-phenyl)-4-(3-pyridyl)-2-pyrimidinamin und 4,0 ml (34,6 mMol) Benzoylchlorid in 320 ml Pyridin werden bei RT unter Stickstoff während 23 Stunden gerührt. Das Reaktionsgemisch wird am HV eingeengt, mit 200 ml Wasser versetzt und nach dem Abkühlen auf 0° abfiltriert. Nach dem Trocknen bei 80° am HV wird das Rohprodukt mit $CH_2Cl_2$-Methanol (95:5) aufgeschlämmt und abfiltriert. Man erhält N-(5-Benzoylamido-2-methyl-phenyl)4-(3-pyridyl)-2-pyrimidinamin. Nach einer chromatographischen Trennung erhält man weitere Mengen dieses Produktes; Smp. 173-176°, $R_f$ = 0,65 ($CHCl_3$:Methanol = 9:1).

Das Ausgangsmaterial erhält man folgendermassen:

Stufe 20.1: Zu einer gelben Suspension von 20,0 g (0,13 Mol) 2-Amino-4-nitro-toluol in 50 ml absolutem Ethanol werden innerhalb von 5 Minuten 9,1 ml (0,13 Mol) 65%ige Salpetersäure zugetropft. Nach dem Abklingen der exothermen Reaktion werden 8,32 g (0,198 Mol) Cyanamid, gelöst in 8,3 ml Wasser, zugegeben. Die braune Reaktionsmischung wird 25 Stunden am Rückfluss gekocht, auf 0° gekühlt und abfiltriert. Nach dem Waschen mit 4mal 100 ml Ethanol-Diethylether (1:1) und Trocknen erhält man 2-Methyl-5-nitrophenyl-guanidin-nitrat; Smp. 219-226°.

Stufe 20.2: Zu einer Lösung von 170 g (0,96 Mol) 3-Dimethylamino-1-(3-pyridyl)-2-propen-1-on in 2,0 Liter Isopropanol werden 248,2 g (0,96 Mol) 2-Methyl-5-nitro-phenylguanidin-nitrat zugegeben. Nach der Zugabe von 42,5 g Natriumhydroxid wird die rötliche Suspension 12 Stunden am Rückfluss gekocht. Nach dem Abkühlen auf 0°, Abfiltrieren, Waschen mit 2,0 Liter Isopropanol und 3mal 400 ml Methanol und Trocknen erhalt man N-(2-Methyl-5-nitro-phenyl)-4-(3-pyridyl)-2-pyrimidinamin, Smp. 195-198°, $R_f$ = 0,68 (Methylenchlorid:Methanol = 9:1).

Stufe 20.3: Eine Suspension von 143,0 g (0,46 Mol) N-(2-Methyl-5-nitro-phenyl)-4-(3-pyridyl)-2-pyrimidinamin in 7,15 Liter Essigsäureethylester wird mit 14,3 g Palladium auf Aktivkohle (10 % Pd) unter einer Wasserstoffatmosphäre bei Normaldruck während 6,5 Stunden gerührt. Die Suspension wird filtriert und das Filtrat am Rotationsverdampfer eingeengt. Das Rohprodukt wird aus Methylenchlorid umkristallisiert. Man erhält N-(5-Amino-2-methylphenyl)-4-(3-pyridyl)-2-pyrimidinamin; Smp. 138-140°, $R_f$ = 0,36 (Methylenchlorid:Methanol = 9:1).

Beispiel 21: Analog Beispiel 20 wird aus 10,68 g (32,8 mMol) 4-(4-Methyl-piperazinomethyl)-benzoylchlorid N-{5-[4-(4-Methyl-piperazino-methyl)-benzoylamido]-2-methylphenyl}-4-(3-pyridyl)-2-pyrimidinamin hergestellt; Smp. 211-213°, $R_f$ = 0,33 (Methylenchlorid:Methanol:25%ige wässerige Ammoniaklösung = 95:5:1).

Beispiel 22: Analog Beispiel 20 wird aus 0,23 ml (1,7 mMol) p-Toluoylchlorid (p-Toluylchlorid) N-[5-(4-Methyl-benzoylamido)-2-methyl-phenyl]-4-(3-pyridyl)-2-pyrimidinamin hergestellt; Smp. 102-106°, $R_f$ = 0,4 (Methylenchlorid:Methanol = 9:1).

Beispiel 23: Analog Beispiel 20 wird aus 330 mg (1,73 mMol) 2-Naphthoylchlorid N-[5-(2-Naphthoylamido)-2-methyl-phenyl]-4-(3-pyridyl)-2-pyrimidinamin hergestellt; Smp. 97-101°, $R_f$ = 0,45 (Methylenchlorid:Methanol = 9:1).

Beispiel 24: Analog Beispiel 20 wird aus 0,22 ml (1,73 mMol) 4-Chlor-benzoylchlorid N-[5-(4-Chlor-benzoylamido)-2-methyl-phenyl]-4-(3-pyridyl)-2-pyrimidinamin synthetisiert; Smp. 216-219°, $R_f$ = 0,39 (Methylenchlorid:Methanol = 9:1).

Beispiel 25: Analog Beispiel 20 wird aus 0,28 ml (1,87 mMol) 2-Methoxy-benzoylchlorid N-[5-(2-Methoxy-benzoylamido)-2-methyl-phenyl]-4-(3-pyridyl)-2-pyrimidinamin hergestellt Smp. 88-92°, $R_f$ = 0,45 (Methylenchlorid:Methanol = 9:1).

Beispiel 26: Analog Beispiel 1 erhält man aus 1,0 g (5,68 mMol) 3-Dimethylamino-1-(3-pyridyl)-2-propen-1-on und 1,53 g (5,68 mMol) 3-Trifluormethoxy-phenyl-guanidin-nitrat das N-(3-Trifluormethoxy-phenyl)-4-(3-pyridyl)-2-pyrimidinamin; $R_f$ = 0,7 (Chloroform:Methanol = 9:1).

Das Ausgangsmaterial erhält man folgendermassen:

Stufe 26.1: Analog Stufe 1.1 wird aus 2,0 g (11,3 mMol) 3-Trifluoromethoxy-anilin und 1,4 g (16,6 Mol) Cyanamid (50% in Wasser) das 3-Trifluormethoxy-phenyl-guanidin-nitrat hergestellt; $R_f$ = 0,1 (Methylenchlorid:Methanol:25%ige wässerige Ammoniaklösung = 150:10:1).

Beispiel 27: Analog Beispiel 1 erhält man aus 1,0 g (5,68 mMol) 3-Dimethylamino-1-(3-pyridyl)-2-propen-1-on und 1,78 g (5,68 mMol) 3-(1,1,2,2-Tetrafluoro-ethoxy)-phenyl-guanidin-nitrat das N-(3-[1,1,2,2-Tetrafluoro-ethoxy]-phenyl)-4-(3-pyridyl)-2-pyrimidinamin; $R_f$ = 0,75 (Chloroform:Methanol = 9:1).

Das Ausgangsmaterial erhält man folgendermassen:

Stufe 27.1: Analog Stufe 1.1 wird aus 2,09 g (10 mMol) 3-(1,1,2,2-Tetrafluoro-ethoxy)anilin und 1,26 g (15 Mol) Cyanamid (50% in Wasser) das 3-(1,1,2,2-Tetrafluoro-ethoxy)-phenyl-guanidin-nitrat hergestellt; $R_f$ = 0,15 (Methylenchlorid:Methanol:25%ige wässerige Ammoniaklösung = 150:10:1).

Beispiel 28: Analog Beispiel 1 erhält man aus 1,0 g (5,68 mMol) 3-Dimethylamino-1-(3-pyridyl)-2-propen-1-on und 1,46 g (5,68 mMol) 3-Nitro-5-methyl-phenyl-guanidin-nitrat das N-(3-Nitro-5-methyl-phenyl)-4-(3-pyridyl)-2-pyrimidinamin; $R_f$ = 0,72 (Chloroform:Methanol = 9:1).

Das Ausgangsmaterial erhält man folgendermassen:

Stufe 28.1: Analog Stufe 1.1 wird aus 1,52 g (10 mMol) 3-Nitro-5-methylanilin und 1,26 g (15 Mol) Cyanamid (50% in Wasser) das 3-Nitro-5-methyl-phenyl-guanidin-nitrat hergestellt; $R_f$ = 0,1 (Methylenchlorid:Methanol:25%ige wässerige Ammoniaklösung = 150:10:1).

Beispiel 29: Analog Beispiel 1 erhält man aus 1,0 g (5,68 mMol) 3-Dimethylamino-1-(3-pyridyl)-2-propen-1-on und 1,76 g (5,68 mMol) 3-Nitro-5-trifluoromethyl-phenyl-guanidin-nitrat das N-(3-Nitro-5-trifluoromethyl-phenyl)-4-(3-pyridyl)-2-pyrimidinamin; $R_f$ = 0,8 (Chloroform:Methanol = 9:1).

Das Ausgangsmaterial erhält man folgendermassen:

Stufe 29.1: Analog Stufe 1.1 wird aus 2,06 g (10 mMol) 3-Nitro-5-trifluormethylanilin und 1,26 g (15 Mol) Cyanamid (50% in Wasser) das 3-Nitro-5-trifluormethyl-phenyl-guanidin-nitrat hergestellt; $R_f$ = 0,2 (Methylenchlorid:Methanol:25%ige wässerige Ammoniaklösung = 150:10:1).

Beispiel 30: 200 mg (0,68 Mmol) N-(3-Nitro-phenyl)-4-(3-pyridyl)-2-pyrimidinamin wird in 5 ml Methylenchlorid suspendiert und mit 225 mg (0,71 mMol) 3-Chlor-perbenzoesäure versetzt. Nach 2 Std. werden weitere 10 ml Methylenchlorid zugegeben. Die Suspension wird noch 20 Stunden bei RT gerührt. Filtration und Flash-Chromatographie (Methylenchlorid:Methanol:25%ige wässerige Ammoniaklösung = 90:10:1) des Rückstandes ergibt N-(3-Nitro-phenyl)-4-(N-oxido-3-pyridyl)-2-pyrimidinamin; $R_f$ = 0,4 (Methylenchlorid:Methanol:25%ige wässerige Ammoniaklösung = 90:10:1), Smp. 252-258°.

Beispiel 31: 150 mg (0,39 mMol) N-(3-Benzoylamido-5-methyl-phenyl)-4-(3-pyridyl)2-pyrimidinamin wird in 6 ml Methylenchlorid suspendiert und mit 129 mg (0,41 mMol) 3-Chlorperbenzoesäure versetzt. Nach 22 Stunden wird abfiltriert und der Rückstand mit einer Flash-Chromatographie (Methylenchlorid:Methanol:25%ige wässerige Ammoniaklösung = 90:10:1) gereinigt. Man erhält N-(3-Benzoylamido-5-methyl-phenyl)-4-(N-oxido-3-pyridyl)-2-pyrimidinamin; $R_f$ = 0,3 (Methylenchlorid:Methanol:25%ige wässerige Ammoniaklösung = 90:10:1), Smp. 295-300°.

Beispiel 32: Tabletten, enthaltend 20 mg an Wirkstoff, z.B. eine der in den Beispielen 1-31 beschriebenen Verbindungen der Formel I, werden in folgender Zusammensetzung in üblicher Weise hergestellt:

Zusammensetzung:

| | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung: Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 33: Tabletten enthaltend 1 mg an Wirkstoff, z.B. eine der in den Beispielen 1-31 beschriebenen Verbindungen der Formel I, werden in folgender Zusammensetzung in üblicher Weise hergestellt:

Zusammensetzung:

| | |
|---|---|
| Wirkstoff | 1 mg |
| Weizenstärke | 60 mg |
| Milchzucker | 50mg |
| Kolloidale Kieselsäure | 5 mg |
| Talk | 9 mg |
| Magnesiumstearat | 1 mg |
| | 126 mg |

Herstellung: Der Wirkstoff wird mit einem Teil der Weizenstarke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

Beispiel 34: Kapseln, enthaltend 10 mg an Wirkstoff, z.B. eine der in den Beispielen 1-31 beschriebenen Verbindungen der Formel I, werden wie folgt auf übliche Weise hergestellt:

Zusammensetzung:

| | |
|---|---|
| Wirkstoff | 2500 mg |
| Talkum | 200 mg |
| Kolloidale Kieselsäure | 50 mg |

Herstellung: Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

## Patentansprüche

**1.** N-Phenyl-2-pyrimidinamin-derivate der Formel I,

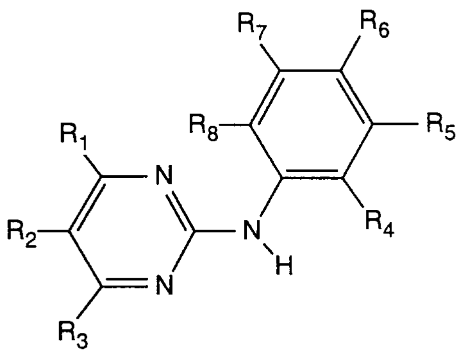

(I)

worin $R_1$ Pyrazinyl, 1-Methyl-1H-pyrrolyl, durch Amino oder Aminoniederalkyl substituiertes Phenyl, worin die Aminogruppe jeweils frei ist oder als Niederalkylamino, Diniederalkylamino, Niederalkanoylamino oder Benzoylamino vorliegt, an einem Fünfringkohlenstoffatom gebundenes 1H-Indolyl oder 1H-Imidazolyl, oder an einem Ringkohlenstoffatom gebundenes, unsubstituiertes oder durch Niederalkyl substituiertes Pyridyl, welches am Stickstoff unsubstituiert oder durch Sauerstoff substituiert ist, bedeutet, $R_2$ und $R_3$ unabhängig voneinander je Wasserstoff oder Niederalkyl bedeuten, einer oder zwei der Reste $R_4$, $R_5$, $R_6$, $R_7$ und $R_8$ je Nitro, fluorsubstituiertes Niederalkoxy oder einen Rest der Formel II bedeuten,

$$-N(R_9)-C(=X)-(Y)_n-R_{10} \quad (II)$$

worin $R_9$ für Wasserstoff oder Niederalkyl, X für Oxo, Thio, Imino, N-Niederalkyl-imino, Hydroximino oder O-Niederalkyl-hydroximino, Y für Sauerstoff oder die Gruppe NH, n für 0 oder 1 und $R_{10}$ für einen aliphatischen Kohlenwasserstoffrest mit 5-22 C-Atomen, für einen Phenyl- oder Naphthylrest, welcher jeweils unsubstituiert oder durch Cyano, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy, Niederalkoxycarbonyl oder durch unsubstituiertes oder substituiertes Niederalkyl substituiert ist, für Phenylniederalkyl, worin der Phenylrest unsubstituiert oder wie vorstehend angegeben substituiert ist, für einen Cycloalkyl- oder Cycloalkenylrest mit bis zu 30 C-Atomen, für Cycloalkyl-niederalkyl oder Cycloalkenyl-niederalkyl mit jeweils bis zu 30 C-Atomen im Cycloalkyl- oder Cycloalkenylteil, für einen monocyclischen Rest mit 5 oder 6 Ringgliedern und 1-3 Ringatomen, ausgewählt aus Stickstoff, Sauerstoff und Schwefel, an welchen ein oder zwei Benzolreste annelliert sein können, oder für Niederalkyl, welches durch einen solchen monocyclischen Rest substituiert ist, steht, und die übrigen der Reste $R_4$, $R_5$, $R_6$, $R_7$ und $R_8$ unabhängig voneinander je Wasserstoff, Niederalkyl, welches unsubstituiert oder durch Amino, Niederalkylamino, Diniederalkylamino, Piperazinyl, Piperidinyl, Pyrrolidinyl oder Morpholinyl substituiert ist, Niederalkanoyl,

Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder Niederalkoxycarbonyl bedeuten, wobei das Präfix "Nieder-" jeweils einen Rest bis und mit 7 C-Atomen bezeichnet, und Salze von solchen Verbindungen mit mindestens einer salzbildenden Gruppe.

**2.** Verbindungen der Formel I nach Anspruch 1, worin einer oder zwei der Reste $R_4$, $R_5$, $R_6$, $R_7$ und $R_8$ je Nitro oder einen Rest der Formel II bedeuten, worin $R_9$ für Wasserstoff oder Niederalkyl, X für Oxo, Thio, Imino, N-Niederalkyl-imino, Hydroximino oder O-Niederalkyl-hydroximino, Y für Sauerstoff oder die Gruppe NH, n für 0 oder 1 und $R_{10}$ für einen aliphatischen Kohlenwasserstoffrest mit 5-22 C-Atomen, für einen Phenyl- oder Naphthylrest, welcher jeweils unsubstituiert oder durch Cyano, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy, Niederalkoxycarbonyl oder durch unsubstituiertes oder substituiertes Niederalkyl substituiert ist, für Phenylniederalkyl, worin der Phenylrest unsubstituiert oder wie vorstehend angegeben substituiert ist, für einen Cycloalkyl- oder Cycloalkenylrest mit bis zu 30 C-Atomen, für Cycloalkyl-niederalkyl oder Cycloalkenyl-niederalkyl mit jeweils bis zu 30 C-Atomen im Cycloalkyl- oder Cycloalkenylteil, für einen monocyclischen Rest mit 5 oder 6 Ringgliedern und 1-3 Ringatomen, ausgewählt aus Stickstoff, Sauerstoff und Schwefel, an welchen ein oder zwei Benzolreste annelliert sein können, oder für Niederalkyl, welches durch einen solchen monocyclischen Rest substituiert ist, steht, und die übrigen der Reste $R_4$, $R_5$, $R_6$, $R_7$ und $R_8$ unabhängig voneinander je Wasserstoff, Niederalkyl, welches unsubstituiert oder durch Amino, Niederalkylamino, Diniederalkylamino, Piperazinyl, Piperidinyl, Pyrrolidinyl oder Morpholinyl substituiert ist, Niederalkanoyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder Niederalkoxycarbonyl bedeuten, und die übrigen Substituenten die im Anspruch 1 genannten Bedeutungen haben, und Salze von solchen Verbindungen mit mindestens einer salzbildenden Gruppe.

**3.** Verbindungen der Formel I nach Anspruch 1, worin $R_1$ Pyrazinyl, 1-Methyl-1H-pyrrolyl, durch Amino oder Aminoniederalkyl substituiertes Phenyl, worin die Aminogruppe jeweils frei, durch ein oder zwei Niederalkylreste alkyliert oder durch Niederalkanoyl oder Benzoyl acyliert ist, an einem Fünfringkohlenstoffatom gebundenes 1H-Indolyl oder 1H-Imidazolyl, oder an einem Ringkohlenstoffatom gebundenes, unsubstituiertes oder durch Niederalkyl substituiertes Pyridyl, welches am Stickstoff unsubstituiert oder durch Sauerstoff substituiert ist, bedeutet, $R_2$ und $R_3$ unabhängig voneinander je Wasserstoff oder Niederalkyl bedeuten, einer oder zwei der Reste $R_4$, $R_5$, $R_6$, $R_7$ und $R_8$ je Nitro, fluorsubstituiertes Niederalkoxy oder einen Rest der Formel II bedeuten, worin $R_9$ für Wasserstoff oder Niederalkyl, X für Oxo, Thio, Imino, N-Niederalkyl-imino, Hydroximino oder O-Niederalkyl-hydroximino, Y für Sauerstoff oder die Gruppe NH, n für 0 oder 1 und $R_{10}$ für einen aliphatischen Kohlenwasserstoffrest mit 5-22 C-Atomen, für einen Phenyl- oder Naphthylrest, welcher jeweils unsubstituiert oder durch Cyano, Niederalkyl, Hydroxyniederalkyl, Amino-niederalkyl, (4-Methyl-piperazinyl)-niederalkyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder durch Niederalkoxycarbonyl substituiert ist, für Phenylniederalkyl, worin der Phenylrest unsubstituiert oder wie vorstehend angegeben substituiert ist, für einen Cycloalkyl- oder Cycloalkenylrest mit bis zu 30 C-Atomen, für Cycloalkyl-niederalkyl oder Cycloalkenyl-niederalkyl mit jeweils bis zu 30 C-Atomen im Cycloalkyl- oder Cycloalkenylteil, für einen monocyclischen Rest mit 5 oder 6 Ringgliedern und 1-3 Ringatomen, ausgewählt aus Stickstoff, Sauerstoff und Schwefel, an welchen ein oder zwei Benzolreste annelliert sein können, oder für Niederalkyl, welches durch einen solchen monocyclischen Rest substituiert ist, steht, und die übrigen der Reste $R_4$, $R_5$, $R_6$, $R_7$ und $R_8$ unabhängig voneinander je Wasserstoff, Niederalkyl, welches unsubstituiert oder durch Amino, Niederalkylamino, Diniederalkylamino, Piperazinyl, Piperidinyl, Pyrrolidinyl oder durch Morpholinyl substituiert ist, Niederalkanoyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder Niederalkoxycarbonyl bedeuten, und Salze von solchen Verbindungen mit mindestens einer salzbildenden Gruppe.

**4.** Verbindungen der Formel I nach Anspruch 1, worin $R_1$ an einem Ringkohlenstoffatom gebundenes Pyridyl, welches am Stickstoff unsubstituiert oder durch Sauerstoff substituiert ist, bedeutet, $R_2$ und $R_3$ je Wasserstoff bedeuten, $R_4$ Wasserstoff oder Niederalkyl bedeutet, $R_5$ Wasserstoff, Niederalkyl oder fluorsubstituiertes Niederalkoxy bedeutet, $R_6$ Wasserstoff bedeutet, $R_7$ Nitro, fluorsubstituiertes Niederalkoxy oder einen Rest der Formel II bedeutet, worin $R_9$ für Wasserstoff, X für Oxo, n für 0 und $R_{10}$ für einen aliphatischen Kohlenwasserstoffrest mit 5-22 C-Atomen, für einen Phenylrest, welcher unsubstituiert oder durch Cyano, Niederalkyl, (4-Methyl-piperazinyl)-niederalkyl, Niederalkoxy, Halogen oder durch Carboxy substituiert ist, für einen Cycloalkylrest mit bis zu 30 C-Atomen oder für einen monocyclischen Rest mit 5 oder 6 Ringgliedern und 1-3 Schwefelringatomen stehen, und $R_8$ Wasserstoff bedeutet, und pharmazeutisch verwendbare Salze von solchen Verbindungen mit mindestens einer salzbildenden Gruppe.

**5.** Verbindungen der Formel I nach Anspruch 1, worin $R_1$ jeweils an einem Kohlenstoffatom gebundenes Pyridyl oder N-Oxido-pyridyl bedeutet, $R_2$ und $R_3$ je Wasserstoff bedeuten, $R_4$ Wasserstoff oder Niederalkyl bedeutet, $R_5$ Wasserstoff, Niederalkyl oder Trifluormethyl bedeutet, $R_6$ Wasserstoff bedeutet, $R_7$ für Nitro, fluorsubstituiertes Niederalkoxy oder einen Rest der Formel II steht, worin $R_9$ Wasserstoff, X Oxo, n die Zahl 0 und $R_{10}$ an einem Kohlenstoffatom gebundenes Pyridyl, unsubstituiertes oder durch Halogen, Cyano, Niederalkoxy, Carboxy, Niederalkyl oder 4-Methyl-piperazinyl-methyl substituiertes Phenyl, $C_5$-$C_7$-Alkyl, Thienyl, 2-Naphthyl oder Cyclohexyl bedeuten, und $R_8$ Wasserstoff bedeutet, und pharmazeutisch verwendbare Salze von solchen Verbindungen mit mindestens einer salzbildenden Gruppe.

**6.** Verbindungen nach einem der Ansprüche 1-5 der Formel I, worin $R_4$ und $R_8$ je für Wasserstoff stehen und die übrigen Substituenten die in dem jeweiligen übergeordneten Anspruch genannten Bedeutungen haben, und pharmazeutisch verwendbare Salze von solchen Verbindungen mit mindestens einer salzbildenden Gruppe.

**7.** Verbindungen nach einem der Ansprüche 1-5 der Formel I, worin mindestens einer der Reste $R_4$ und $R_8$ für Niederalkyl steht und die übrigen Substituenten die in dem jeweiligen übergeordneten Anspruch genannten Bedeutungen haben, und pharmazeutisch verwendbare Salze von solchen Verbindungen mit mindestens einer salzbildenden Gruppe.

**8.** Verbindungen nach Anspruch 1 der Formel I, worin $R_1$ an einem Kohlenstoffatom gebundenes Pyridyl bedeutet, $R_2$, $R_3$, $R_4$, $R_5$, $R_6$ und $R_8$ je Wasserstoff bedeuten und $R_7$ für Nitro oder einen Rest der Formel II steht, worin $R_9$ Wasserstoff, X Oxo, n die Zahl 0 und $R_{10}$ an einem Kohlenstoffatom gebundenes Pyridyl, unsubstituiertes oder durch Fluor, Chlor, Cyano, Niederalkoxy, Carboxy, Niederalkyl oder 4-Methyl-piperazinyl-methyl substituiertes Phenyl, $C_5$-$C_7$-Alkyl, Thienyl oder Cyclohexyl bedeuten, und pharmazeutisch verwendbare Salze davon.

**9.** Eine Verbindung nach Anspruch 1 der Formel I, ausgewählt aus

N-(3-Nitro-phenyl)-4-(3-pyridyl)-2-pyrimidinamin,
N-[3-(4-Chlorbenzoylamido)-phenyl]-4-(3-pyridyl)-2-pyrimidinamin,
N-(3-Benzoylamido-phenyl)-4-(3-pyridyl)-2-pyrimidinamin,
N-[3-(2-Pyridyl)carboxamido-phenyl]-4-(3-pyridyl)-2-pyrimidinamin,
N-[3-(3-Pyridyl)carboxamido-phenyl]-4-(3-pyridyl)-2-pyrimidinamin,
N-[3-(4-Pyridyl)carboxamido-phenyl]-4-(3-pyridyl)-2-pyrimidinamin,
N-(3-Pentafluor-benzoylamido-phenyl)-4-(3-pyridyl)-2-pyrimidinamin,
N-[3-(2-carboxy-benzoylamido)-phenyl]-4-(3-pyridyl)-2-pyrimidinamin,
N-(3-n-Hexanoylamido-phenyl)-4-(3-pyridyl)-2-pyrimidinamin,
N-(3-Nitro-phenyl)-4-(2-pyridyl)-2-pyrimidinamin,
N-(3-Nitro-phenyl)-4-(4-pyridyl)-2-pyrimidinamin,
N-[3-(2-Methoxy-benzoylamido)-phenyl]-4-(3-pyridyl)-2-pyrimidinamin,
N-[3-(4-Fluor-benzoylamido)-phenyl]-4-(3-pyridyl)-2-pyrimidinamin,
N-[3-(4-Cyano-benzoylamido)-phenyl]-4-(3-pyridyl)-2-pyrimidinamin,
N-[3-(2-Thienylcarboxamido)-phenyl]-4-(3-pyridyl)-2-pyrimidinamin,
N-(3-Cyclohexylcarboxamido-phenyl)-4-(3-pyridyl)-2-pyrimidinamin,
N-[3-(4-Methyl-benzoylamido)-phenyl]-4-(3-pyridyl)-2-pyrimidinamin,
N-[3-(4-Chlor-benzoylamido)-phenyl]-4-(4-pyridyl)-2-pyrimidinamin,
N-{3-[4-(4-Methyl-piperazinomethyl)-benzoylamido]-phenyl ]-4-(3-pyridyl)-2-pyrimidinamin,
N-[5-(4-Methyl-benzoylamido)-2-methyl-phenyl]-4-(3-pyridyl)-2-pyrimidinamin,
N-[5-(2-Naphthoylamido)-2-methyl-phenyl]-4-(3-pyridyl)-2-pyrimidinamin,
N-[5-(4-Chlor-benzoylamido)-2-methyl-phenyl]-4-(3-pyridyl)-2-pyrimidinamin,
N-[5-(2-Methoxy-benzoylamido)-2-methyl-phenyl]-4-(3-pyridyl)-2-pyrimidinamin,
N-(3-Trifluormethoxy-phenyl)-4-(3-pyridyl)-2-pyrimidinamin,
N-(3-[1,1,2,2-Tetrafluoro-ethoxy]-phenyl)-4-(3-pyridyl)-2-pyrimidinamin,
N-(3-Nitro-5-methyl-phenyl)-4-(3-pyridyl)-2-pyrimidinamin,
N-(3-Nitro-5-trifluoromethyl-phenyl)-4-(3-pyridyl)-2-pyrimidinamin,
N-(3-Nitro-phenyl)-4-(N-oxido-3-pyridyl)-2-pyrimidinamin,
N-(3-Benzoylamido-5-methyl-phenyl)-4-(N-oxido-3-pyridyl)-2-pyrimidinamin und den pharmazeutisch verwendbaren Salzen dieser Verbindungen mit mindestens einer salzbildenden Gruppe.

**10.** N-(5-Benzoylamido-2-methyl-phenyl)-4-(3-pyridyl)-2-pyrimidinamin der Formel I nach Anspruch 1.

**11.** N-{5-[4-(4-Methyl-piperazino-methyl)-benzoylamido]-2-methyl-phenyl}-4-(3-pyridyl)-2-pyrimidinamin der Formel I nach Anspruch 1 oder ein pharmazeutisch verwendbares Säureadditionssalz davon.

**12.** Eine Verbindung der Formel I gemäss einem der Ansprüche 1-11 oder ein pharmazeutisch verwendbares Salz einer solchen Verbindung mit mindestens einer salzbildenden Gruppe zur Anwendung in einem Verfahren zur therapeutischen Behandlung des menschlichen oder tierischen Körpers.

**13.** Pharmazeutische Zusammensetzung, enthaltend eine Verbindung der Formel I gemäss einem der Ansprüche 1-11 oder ein pharmazeutisch verwendbares Salz einer solchen Verbindung mit mindestens einer salzbildenden Gruppe zusammen mit pharmazeutischem Trägermaterial.

**14.** Verwendung einer Verbindung der Formel I gemäss einem der Ansprüche 1-11 oder eines pharmazeutisch verwendbaren Salzes einer solchen Verbindung mit mindestens einer salzbildenden Gruppe zur Herstellung von pharmazeutischen Zusammensetzungen zur Anwendung zur Chemotherapie von Tumoren.

**15.** Verwendung einer Verbindung der Formel I gemäss einem der Ansprüche 1-11 oder eines pharmazeutisch verwendbaren Salzes einer solchen Verbindung mit mindestens einer salzbildenden Gruppe zur Herstellung von pharmazeutischen Zusammensetzungen zur Anwendung zur Behandlung von Atherosklerose.

**16.** Verfahren zur Herstellung einer Verbindung der Formel I gemäss Anspruch 1 oder eines Salzes einer solchen Verbindung mit mindestens einer salzbildenden Gruppe, dadurch gekennzeichnet, dass man

a) eine Verbindung der Formel III,

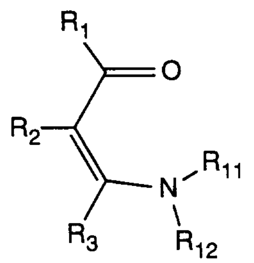

(III)

worin $R_{11}$ und $R_{12}$ unabhängig voneinander je für Niederalkyl stehen und $R_1$, $R_2$ und $R_3$ die obengenannten Bedeutungen haben, wobei in einer Verbindung der Formel III vorhandene funktionelle Gruppen mit Ausnahme der an der Reaktion teilnehmenden Gruppen erforderlichenfalls in geschützter Form vorliegen, oder ein Salz einer solchen Verbindung mit einer Verbindung der Formel IV,

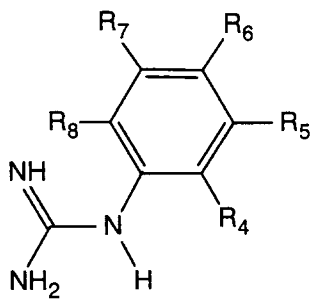

(IV)

worin die Substituenten die obengenannten Bedeutungen haben, wobei in einer Verbindung der Formel IV vorhandene funktionelle Gruppen mit Ausnahme der an der Reaktion teilnehmenden Guanidinogruppe erforderlichenfalls in geschützter Form vorliegen, oder mit einem Salz einer solchen Verbindung umsetzt, und vorhandene Schutzgruppen abspaltet, oder

b) zur Herstellung einer Verbindung der Formel I, worin die Reste $R_4$, $R_5$, $R_6$, $R_7$ und $R_8$ die obengenannten

Bedeutungen mit Ausnahme von Nitro und fluorsubstituiertem Niederalkoxy haben, eine Verbindung der Formel V,

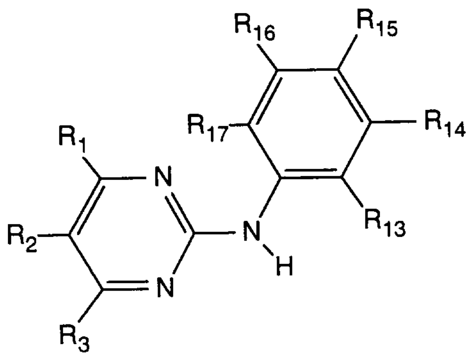

(V)

worin einer oder zwei der Reste $R_{13}$, $R_{14}$, $R_{15}$, $R_{16}$ und $R_{17}$ je $R_9$-Amino und die übrigen der Reste $R_{13}$, $R_{14}$, $R_{15}$, $R_{16}$ und $R_{17}$ unabhängig voneinander je Wasserstoff, Niederalkyl, welches unsubstituiert oder durch Amino, Niederalkylamino, Diniederalkylamino, Piperazinyl, Piperidinyl, Pyrrolidinyl oder Morpholinyl substituiert ist, Niederalkanoyl, Trifluormethyl, Hydroxy, Niederalkoxy, Niederalkanoyloxy, Halogen, Amino, Niederalkylamino, Diniederalkylamino, Niederalkanoylamino, Benzoylamino, Carboxy oder Niederalkoxycarbonyl bedeuten, und $R_1$, $R_2$, $R_3$ und $R_9$ die obengenannten Bedeutungen haben, wobei in einer Verbindung der Formel V vorhandene funktionelle Gruppen mit Ausnahme der an der Reaktion teilnehmenden $R_9$-Aminogruppe(n) erforderlichenfalls in geschützter Form vorliegen, mit einer Verbindung der Formel VI,

$$HO\text{-}C(=X)\text{-}(Y)_n\text{-}R_{10} \qquad (VI)$$

worin die Substituenten und Symbole die obengenannten Bedeutungen haben, wobei in einer Verbindung der Formel VI vorhandene funktionelle Gruppen mit Ausnahme der an der Reaktion teilnehmenden HO-C(=X)-Gruppe erforderlichenfalls in geschützter Form vorliegen, oder mit einem reaktionsfähigen Derivat einer Verbindung der Formel VI umsetzt und vorhandene Schutzgruppen abspaltet, oder

c) zur Herstellung einer Verbindung der Formel I, worin $R_1$ Pyridyl bedeutet, welches am Stickstoff durch Sauerstoff substituiert ist, und worin die übrigen Substituenten und Symbole die obengenannten Bedeutungen haben, eine Verbindung der Formel I, worin $R_1$ Pyridyl bedeutet, mit einem geeigneten Oxidationsmittel in die N-Oxido-Verbindung überführt,
und wenn erwünscht, eine nach einem der Verfahren a bis c erhaltene Verbindung der Formel I in ihr Salz überführt, oder ein erhaltenes Salz einer Verbindung der Formel I in die freie Verbindung umwandelt.

## Claims

**1.** An N-phenyl-2-pyrimidine-amine derivative of formula I

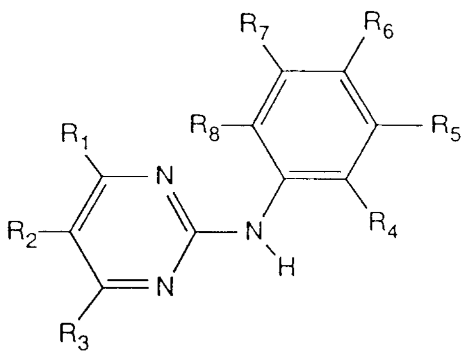

(I),

wherein

$R_1$ is pyrazinyl, 1-methyl-1H-pyrrolyl, amino- or amino-lower alkyl-substituted phenyl wherein the amino group in each case is free or is in the form of lower alkylamino, dilower alkylamino, lower alkanoylamino or benzoylamino, 1H-indolyl or 1H-imidazolyl bonded at a five-membered ring carbon atom, or unsubstituted or lower alkyl-substituted pyridyl bonded at a ring carbon atom and unsubstituted or substituted at the nitrogen atom by oxygen,

$R_2$ and $R_3$ are each independently of the other hydrogen or lower alkyl, one or two of the radicals $R_4$, $R_5$, $R_6$, $R_7$ and $R_8$ are each nitro, fluoro-substituted lower alkoxy or a radical of formula II

$$-N(R_9)-C(=X)-(Y)_n-R_{10} \quad (II),$$

wherein

$R_9$ is hydrogen or lower alkyl,

X is oxo, thio, imino, N-lower alkyl-imino, hydroximino or O-lower alkyl-hydroximino,

Y is oxygen or the group NH,

n is 0 or 1 and

$R_{10}$ is an aliphatic hydrocarbon radical having 5-22 carbon atoms, a phenyl or naphthyl radical each of which is unsubstituted or substituted by cyano, trifluoromethyl, hydroxy, lower alkoxy, lower alkanoyloxy, halogen, amino, lower alkylamino, di-lower alkylamino, lower alkanoylamino, benzoylamino, carboxy, lower alkoxycarbonyl or by unsubstituted or substituted lower alkyl, or phenyl-lower alkyl wherein the phenyl radical is unsubstituted or substituted as indicated above, a cycloalkyl or cycloalkenyl radical having up to 30 carbon atoms, cycloalkyl-lower alkyl or cycloalkenyl-lower alkyl each having up to 30 carbon atoms in the cycloalkyl or cycloalkenyl moiety, a monocyclic radical having 5 or 6 ring members and 1-3 ring atoms selected from nitrogen, oxygen and sulfur, to which radical one or two benzene radicals may be fused, or lower alkyl substituted by such a monocyclic radical,

and the remaining radicals $R_4$, $R_5$, $R_6$, $R_7$ and $R_8$ are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl,

the term "lower" in each case denoting a radical having up to and including 7 carbon atoms, or a salt of such a compound having at least one salt-forming group.

**2.** A compound of formula I according to claim 1, wherein one or two of the radicals $R_4$, $R_5$, $R_6$, $R_7$ and $R_8$ are each nitro or a radical of formula II wherein

$R_9$ is hydrogen or lower alkyl,

X is oxo, thio, imino, N-lower alkyl-imino, hydroximino or O-lower alkyl-hydroximino,

Y is oxygen or the group NH,

n is 0 or 1 and

$R_{10}$ is an aliphatic hydrocarbon radical having 5-22 carbon atoms, a phenyl or naphthyl radical each of which is unsubstituted or substituted by cyano, trifluoromethyl, hydroxy, lower alkoxy, lower alkanoyloxy, halogen, amino, lower alkylamino, di-lower alkylamino, lower alkanoylamino, benzoylamino, carboxy, lower alkoxycarbonyl or by unsubstituted or substituted lower alkyl, or phenyl-lower alkyl wherein the phenyl radical is unsubstituted or substituted as indicated above, a cycloalkyl or cycloalkenyl radical having up to 30 carbon atoms, cycloalkyl-lower alkyl or cycloalkenyl-lower alkyl each having up to 30 carbon atoms in the cycloalkyl or cycloalkenyl moiety, a monocyclic radical having 5 or 6 ring members and 1-3 ring atoms selected from nitrogen, oxygen and sulfur, to which radical one or two benzene radicals may be fused, or lower alkyl substituted by such a monocyclic radical,

and the remaining radicals $R_4$, $R_5$, $R_6$, $R_7$ and $R_8$ are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl,

and the remaining substituents are as defined in claim 1, or a salt of such a compound having at least one salt-forming group.

**3.** A compound of formula I according to claim 1, wherein

$R_1$ is pyrazinyl, 1-methyl-1H-pyrrolyl, amino- or amino-lower alkyl-substituted phenyl wherein the amino group in each case is free, alkylated by one or two lower alkyl radicals or acylated by lower alkanoyl or by benzoyl, 1H-indolyl or 1H-imidazolyl bonded at a five-membered ring carbon atom, or unsubstituted or lower alkyl-substituted pyridyl bonded at a ring carbon atom and unsubstituted or substituted at the nitrogen atom by oxygen,
$R_2$ and $R_3$ are each independently of the other hydrogen or lower alkyl,
one or two of the radicals $R_4$, $R_5$, $R_6$, $R_7$ and $R_8$ are each nitro, fluoro-substituted lower alkoxy or a radical of formula II wherein
$R_9$ is hydrogen or lower alkyl,
X is oxo, thio, imino, N-lower alkyl-imino, hydroximino or O-lower alkyl-hydroximino,
Y is oxygen or the group NH,
n is 0 or 1 and
$R_{10}$ is an aliphatic hydrocarbon radical having 5-22 carbon atoms, a phenyl or naphthyl radical each of which is unsubstituted or substituted by cyano, lower alkyl, hydroxy-lower alkyl, amino-lower alkyl, (4-methyl-piperazinyl)-lower alkyl, trifluoromethyl, hydroxy, lower alkoxy, lower alkanoyloxy, halogen, amino, lower alkylamino, di-lower alkylamino, lower alkanoylamino, benzoylamino, carboxy or by lower alkoxycarbonyl, or phenyl-lower alkyl wherein the phenyl radical is unsubstituted or substituted as indicated above, a cycloalkyl or cycloalkenyl radical having up to 30 carbon atoms, cycloalkyl-lower alkyl or cycloalkenyl-lower alkyl each having up to 30 carbon atoms in the cycloalkyl or cycloalkenyl moiety, a monocyclic radical having 5 or 6 ring members and 1-3 ring atoms selected from nitrogen, oxygen and sulfur, to which radical one or two benzene radicals may be fused, or lower alkyl substituted by such a monocyclic radical,
and the remaining radicals $R_4$, $R_5$, $R_6$, $R_7$ and $R_8$ are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl,
or a salt of such a compound having at least one salt-forming group.

**4.** A compound of formula I according to claim 1, wherein

$R_1$ is pyridyl bonded at a ring carbon atom and unsubstituted or substituted at the nitrogen atom by oxygen,
$R_2$ and $R_3$ are each hydrogen,
$R_4$ is hydrogen or lower alkyl,
$R_5$ is hydrogen, lower alkyl or fluoro-substituted lower alkoxy,
$R_6$ is hydrogen, $R_7$ is nitro, fluoro-substituted lower alkoxy or a radical of formula II wherein
$R_9$ is hydrogen,
X is oxo,
n is 0 and
$R_{10}$ is an aliphatic hydrocarbon radical having 5-22 carbon atoms, a phenyl radical that is unsubstituted or substituted by cyano, lower alkyl, (4-methyl-piperazinyl)-lower alkyl, lower alkoxy, halogen or by carboxy, a cycloalkyl radical having up to 30 carbon atoms or a monocyclic radical having 5 or 6 ring members and 1-3 sulfur ring atoms, and
$R_8$ is hydrogen,
or a pharmaceutically acceptable salt of such a compound having at least one salt-forming group.

**5.** A compound of formula I according to claim 1, wherein

$R_1$ is pyridyl or N-oxido-pyridyl each of which is bonded at a carbon atom,
$R_2$ and $R_3$ are each hydrogen,
$R_4$ is hydrogen or lower alkyl,
$R_5$ is hydrogen, lower alkyl or trifluoromethyl,
$R_6$ is hydrogen,
$R_7$ is nitro, fluoro-substituted lower alkoxy or a radical of formula II wherein
$R_9$ is hydrogen,

X is oxo,
n is the number 0 and
$R_{10}$ is pyridyl bonded at a carbon atom, phenyl that is unsubstituted or substituted by halogen, cyano, lower alkoxy, carboxy, lower alkyl or by 4-methyl-piperazinyl-methyl, or $C_5$-$C_7$-alkyl, thienyl, 2-naphthyl or cyclohexyl, and
$R_8$ is hydrogen,
or a pharmaceutically acceptable salt of such a compound having at least one salt-forming group.

**6.** A compound according to any one of claims 1 to 5 of formula I, wherein $R_4$ and $R_8$ are each hydrogen and the remaining substituents are as defined in the respective generic claim, or a pharmaceutically acceptable salt of such a compound having at least one salt-forming group.

**7.** A compound according to any one of claims 1 to 5 of formula I, wherein at least one of the radicals $R_4$ and $R_8$ is lower alkyl, and the remaining substituents are as defined in the respective generic claim, or a pharmaceutically acceptable salt of such a compound having at least one salt-forming group.

**8.** A compound according to claim 1 of formula I, wherein

$R_1$ is pyridyl bonded at a carbon atom,
$R_2$, $R_3$, $R_4$, $R_5$, $R_6$ and $R_8$ are each hydrogen and
$R_7$ is nitro or a radical of formula II wherein
$R_9$ is hydrogen,
X is oxo,
n is the number 0 and
$R_{10}$ is pyridyl bonded at a carbon atom, phenyl that is unsubstituted or substituted by fluorine, chlorine, cyano, lower alkoxy, carboxy, lower alkyl or by 4-methyl-piperazinyl-methyl, or $C_5$-$C_7$alkyl, thienyl or cyclohexyl,
or a pharmaceutically acceptable salt thereof.

**9.** A compound according to claim 1 of formula I selected from

N-(3-nitro-phenyl)-4-(3-pyridyl)-2-pyrimidine-amine,
N-[3-(4-chlorobenzoylamido)-phenyl]-4-(3-pyridyl)-2-pyrimidine-amine,
N-(3-benzoylamido-phenyl)-4-(3-pyridyl)-2-pyrimidine-amine,
N-[3-(2-pyridyl)carboxamido-phenyl]-4-(3-pyridyl)-2-pyrimidine-amine,
N-[3-(3-pyridyl)carboxamido-phenyl]-4-(3-pyridyl)-2-pyrimidine-amine,
N-[3-(4-pyridyl)carboxamido-phenyl]-4-(3-pyridyl)-2-pyrimidine-amine,
N-(3-pentafluoro-benzoylamido-phenyl)-4-(3-pyridyl)-2-pyrimidine-amine,
N-[3-(2-carboxy-benzoylamido)-phenyl]-4-(3-pyridyl)-2-pyrimidine-amine,
N-(3-n-hexanoylamido-phenyl)-4-(3-pyridyl)-2-pyrimidine-amine,
N-(3-nitro-phenyl)-4-(2-pyridyl)-2-pyrimidine-amine,
N-(3-nitro-phenyl)-4-(4-pyridyl)-2-pyrimidine-amine,
N-[3-(2-methoxy-benzoylamido)-phenyl]-4-(3-pyridyl)-2-pyrimidine-amine,
N-[3-(4-fluoro-benzoylamido)-phenyl]-4-(3-pyridyl)-2-pyrimidine-amine,
N-[3-(4-cyano-benzoylamido)-phenyl]-4-(3-pyridyl)-2-pyrimidine-amine,
N-[3-(2-thienylcarboxamido)-phenyl]-4-(3-pyridyl)-2-pyrimidine-amine,
N-(3-cyclohexylcarboxamido-phenyl)-4-(3-pyridyl)-2-pyrimidine-amine,
N-[3-(4-methyl-benzoylamido)-phenyl]-4-(3-pyridyl)-2-pyrimidine-amine,
N-[3-(4-chloro-benzoylamido)-phenyl]-4-(4-pyridyl)-2-pyrimidine-amine,
N-{3-[4-(4-methyl-piperazinomethyl)-benzoylamido]-phenyl}-4-(3-pyridyl)-2-pyrimidine-amine,
N-[5-(4-methyl-benzoylamido)-2-methyl-phenyl]-4-(3-pyridyl)-2-pyrimidine-amine,
N-[5-(2-naphthoylamido)-2-methyl-phenyl]-4-(3-pyridyl)-2-pyrimidine-amine
N-[5-(4-chloro-benzoylamido)-2-methyl-phenyl]-4-(3-pyridyl)-2-pyrimidine-amine,
N-[5-(2-methoxy-benzoylamido)-2-methyl-phenyl]-4-(3-pyridyl)-2-pyrimidine-amine,
N-(3-torifluoromethoxy-phenyl)-4-(3-pyridyl)-2-pyrimidine-amine,
N-(3-[1,1,2,2-tetrafluoro-ethoxy]-phenyl)-4-(3-pyridyl)-2-pyrimidine-amine
N-(3-nitro-5-methyl-phenyl)-4-(3-pyridyl)-2-pyrimidine-amine,
N-(3-nitro-5-trifluoromethyl-phenyl)-4-(3-pyridyl)-2-pyrimidine-amine,

N-(3-nitro-phenyl)-4-(N-oxido-3-pyridyl)-2-pyrimidine-amine,
N-(3-benzoylamido-5-methyl-phenyl)-4-(N-oxido-3-pyridyl)-2-pyrimidine-amine,
or a pharmaceutically acceptable salt of such a compound having at least one salt-forming group.

**10.** N-(5-Benzoylamido-2-methyl-phenyl)-4-(3-pyridyl)-2-pyrimidine-amine of formula I according to claim 1.

**11.** N-{5-[4-(4-Methyl-piperazino-methyl)-benzoylamido]-2-methyl-phenyl}-4-(3-pyridyl)-2-pyrimidine-amine of formula I according to claim 1 or a pharmaceutically acceptable acid addition salt thereof.

**12.** A compound of formula I according to any one of claims 1 to 11 or a pharmaceutically acceptable salt of such a compound having at least one salt-forming group for use in a method for the therapeutic treatment of the human or animal body.

**13.** A pharmaceutical composition comprising a compound of formula I according to any one of claims 1 to 11 or a pharmaceutically acceptable salt of such a compound having at least one salt-forming group together with a pharmaceutical carrier.

**14.** The use of a compound of formula I according to any one of claims 1 to 11 or a pharmaceutically acceptable salt of such a compound having at least one salt-forming group for the preparation of pharmaceutical compositions for use in the chemotherapy of tumours.

**15.** The use of a compound of formula I according to any one of claims 1 to 11 or a pharmaceutically acceptable salt of such a compound having at least one salt-forming group for the preparation of pharmaceutical compositions for use in the treatment of atherosclerosis.

**16.** A process for the preparation of a compound of formula I according to claim 1 or of a salt of such a compound having at least one salt-forming group, which comprises

a) reacting a compound of formula III

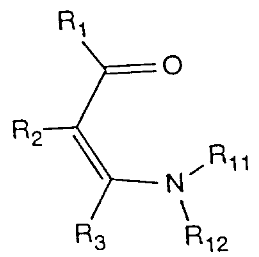

(III),

wherein $R_{11}$ and $R_{12}$ are each independently of the other lower alkyl and $R_1$, $R_2$ and $R_3$ are as defined above, functional groups present in a compound of formula III, with the exception of the groups participating in the reaction, being if necessary in protected form, or a salt of such a compound, with a compound of formula IV

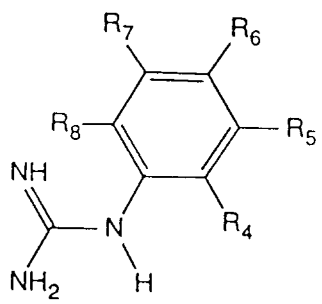

(IV),

wherein the substituents are as defined above, functional groups present in a compound of formula IV, with the

exception of the guanidino group participating in the reaction, being if necessary in protected form, or with a salt of such a compound, and removing any protecting groups present, or

b) for the preparation of a compound of formula I wherein the radicals $R_4$, $R_5$, $R_6$, $R_7$ and $R_8$ are as defined above with the exception of nitro and fluoro-substituted lower alkoxy, reacting a compound of formula V

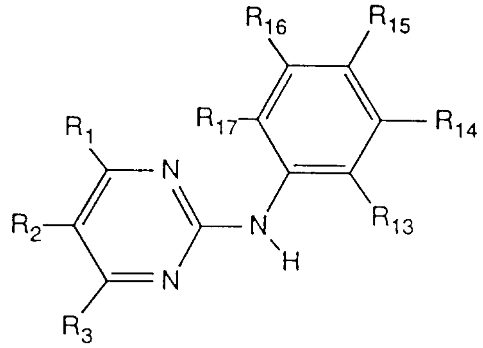

(V),

wherein one or two of the radicals $R_{13}$, $R_{14}$, $R_{15}$, $R_{16}$ and $R_{17}$ are each $R_9$-amino and the remaining radicals $R_{13}$, $R_{14}$, $R_{15}$, $R_{16}$ and $R_{17}$ are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl, and $R_1$, $R_2$, $R_3$ and $R_9$ are as defined above, functional groups present in a compound of formula V, with the exception of the $R_9$-amino group(s) participating in the reaction, being if necessary in protected form, with a compound of formula VI

$$HO\text{-}C(=X)\text{-}(Y)_n\text{-}R_{10} \quad (VI),$$

wherein the substituents and symbols are as defined above, functional groups present in a compound of formula VI, with the exception of the HO-C(=X) group participating in the reaction, being if necessary in protected form, or with a reactive derivative of a compound of formula VI, and removing any protecting groups present, or

c) for the preparation of a compound of formula I wherein $R_1$ is pyridyl substituted at the nitrogen atom by oxygen, and wherein the other substituents and symbols are as defined above, converting a compound of formula I wherein $R_1$ is pyridyl into the N-oxido compound with a suitable oxidising agent,
and, if desired, converting a compound of formula I obtainable by any one of processes a to c into its salt, or converting an obtainable salt of a compound of formula I into the free compound.

## Revendications

**1.** Un dérivé de la N-phényl-2-pyrimidine-amine de formule I,

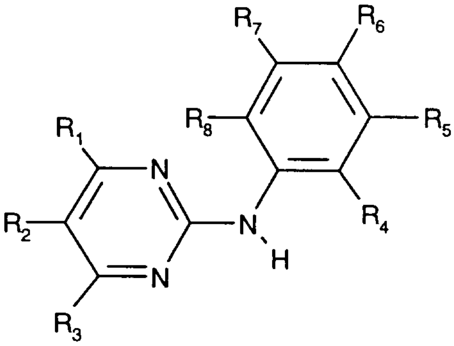

(I)

où $R_1$ signifie un groupe pyrazinyle, 1-méthyl-1-H-pyrrolyle, phényle substitué par un groupe amino ou amino-alkyle inférieur, où à chaque fois le groupe amino est libre ou se présente sous forme d'un groupe alkyl inférieur-amino, di-alkyl inférieur-amino, alcanoyl inférieur-amino ou benzoylamino, un groupe 1H-indolyle ou 1H-imidazolyle lié à un atome de carbone du cycle à 5 chaînons, ou un groupe pyridyle lié à un atome de carbone du cycle, non substitué ou substitué par un groupe alkyle inférieur qui est non substitué sur l'atome d'azote ou substitué par de l'oxygène, $R_2$ et $R_3$ indépendamment l'un de l'autre, signifient chacun l'hydrogène ou un groupe alkyle inférieur, un ou deux des restes $R_4$, $R_5$, $R_6$, $R_7$ et $R_8$ signifient chacun un groupe nitro, alcoxy inférieur substitué par du fluor ou un reste de formule II,

$$-N(R_9)-C(=X)-(Y)_n-R_{10} \qquad (II)$$

où $R_9$ signifie l'hydrogène ou un groupe alkyle inférieur, X signifie un groupe oxo, thio, imino, N-alkyl inférieur-imino, hydroximino ou O-alkyl inférieur-hydroximino, Y signifie l'oxygène ou le groupe NH, n signifie 0 ou 1 et $R_{10}$ signifie un reste hydrocarboné aliphatique ayant 5-22 atomes de carbone, signifie un reste phényle ou naphtyle, qui est à chaque fois non substitué ou substitué par un groupe cyano, trifluorométhyle, hydroxy, alcoxy inférieur, alcanoyloxy inférieur, un halogène, un groupe amino, alkyl inférieur-amino, di-alkyl inférieur-amino, alcanoyl inférieur-amino, benzoylamino, carboxy, alcoxy inférieur-carbonyle ou est substitué par un groupe alkyle inférieur non substitué ou substitué, signifie un groupe phényl-alkyle inférieur, où le reste phényle est non substitué ou substitué comme indiqué plus haut, signifie un reste cycloalkyle ou cycloalcényle ayant jusqu'à 30 atomes de carbone, signifie un groupe cycloalkyl-alkyle inférieur ou cycloalcényl-alkyle inférieur ayant à chaque fois jusqu'à 30 atomes de carbone dans la partie cycloalkyle ou cycloalcényle, signifie un reste monocyclique ayant 5 ou 6 chaînons dans le cycle et 1-3 atomes dans le cycle choisi parmi l'azote, l'oxygène et le soufre, sur lequel un ou deux restes benzène peuvent être condensés, ou signifie un groupe alkyle inférieur qui est substitué par un tel reste monocyclique et les autres restes $R_4$, $R_5$, $R_6$, $R_7$ et $R_8$, indépendamment les uns des autres, signifient chacun l'hydrogène, un groupe alkyle inférieur qui est non substitué ou substitué par un groupe amino, alkyl inférieur-amino, di-alkyl inférieur-amino, pipérazinyle, pipéridinyle, pyrrolidinyle ou morpholinyle, alcanoyle inférieur, trifluorométhyle, hydroxy, alcoxy inférieur, alcanoyloxy inférieur, un halogène, un groupe amino, alkyl inférieur-amino, di-alkyl inférieur-amino, alcanoyl inférieur-amino, benzoylamino, carboxy ou alcoxy inférieur-carbonyle, où le préfixe "inférieur" signifie à chaque fois un reste ayant jusqu'à 7 atomes de carbone, et les sels de tels composés avec au moins un groupe formant un sel.

**2.** Les composés de formule I selon la revendication 1, où un ou deux des restes $R_4$, $R_5$, $R_6$, $R_7$ et $R_8$ signifient chacun un groupe nitro ou un reste de formule II, où $R_9$ signifie l'hydrogène ou un groupe alkyle inférieur, X signifie un groupe oxo, thio, imino, N-alkyl inférieur-imino, hydroximino ou O-alkyl inférieur-hydroximino, Y signifie l'oxygène ou le groupe NH, n signifie 0 ou 1 et $R_{10}$ signifie un reste hydrocarboné aliphatique ayant 5-22 atomes de carbone, ou signifie un reste phényle ou naphtyle qui est à chaque fois non substitué ou substitué par un groupe cyano, trifluorométhyle, hydroxy, alcoxy inférieur, alcanoyloxy inférieur, un halogène, un groupe amino, alkyl inférieur-amino, di-alkyl inférieur-amino, alcanoyl inférieur-amino, benzoylamino, carboxy, alcoxy inférieur-carbonyle ou est substitué par un groupe alkyle inférieur non substitué ou substitué, signifie un groupe phényl-alkyle inférieur, où le reste phényle est non substitué ou est substitué comme indiqué plus haut, signifie un reste cycloalkyle ou cycloalcényle ayant jusqu'à 30 atomes de carbone, signifie un groupe cycloalkyl-alkyle inférieur ou cycloalcényl-alkyle inférieur avec à chaque fois jusqu'à 30 atomes de carbone dans la partie cycloalkyle ou cycloalcényle, signifie un reste monocyclique ayant 5 ou 6 chaînons dans le cycle et 1-3 atomes dans le cycle, choisi parmi l'azote, l'oxygène et le soufre, sur lequel un ou deux restes benzène peuvent être condensés, ou signifie un groupe alkyle inférieur, qui

est substitué par un tel reste monocyclique et les autres restes $R_4$, $R_5$, $R_6$, $R_7$ et $R_8$, indépendamment les uns des autres, signifient chacun l'hydrogène, un groupe alkyle inférieur, qui est non substitué ou substitué par un groupe amino, alkyl inférieur-amino, di-alkyl inférieur-amino, pipérazinyle, pipéridinyle, pyrrolidinyle ou morpholinyle, un groupe alcanoyle inférieur, trifluorométhyle, hydroxy, alcoxy inférieur, alcanoyloxy inférieur, un halogène, un groupe amino, alkyl inférieur-amino, di-alkyl inférieur-amino, alcanoyl inférieur-amino, benzoylamino, carboxy ou alcoxy inférieur-carbonyle et les autres substituants ont les significations données dans la revendication 1, et les sels de tels composés avec au moins un groupe formant un sel.

**3.** Les composés de formule I selon la revendication 1, où $R_1$ signifie un groupe pyrazinyle, 1-méthyl-1H-pyrrolyle, phényle substitué par un groupe amino ou amino-alkyle inférieur, où à chaque fois le groupe amino est libre, alkylé par un ou deux restes alkyle inférieur ou acylé par un groupe alcanoyle inférieur ou benzoyle, un groupe 1H-indolyle ou 1H-imidazolyle lié à un atome de carbone du cycle à 5 chaînons, ou un groupe pyridyle lié à un atome de carbone du cycle, non substitué ou substitué par un groupe alkyle inférieur, qui est non substitué sur l'azote ou substitué par de l'oxygène, $R_2$ et $R_3$ , indépendamment l'un de l'autre, signifient chacun l'hydrogène ou un groupe alkyle inférieur, un ou deux des restes $R_4$, $R_5$, $R_6$, $R_7$ et $R_8$ signifient chacun un groupe nitro, alcoxy inférieur substitué par du fluor ou un reste de formule II, où $R_9$ signifie l'hydrogène ou un groupe alkyle inférieur, X signifie oxo, thio, imino, N-alkyl inférieur-imino, hydroximino ou O-alkyl inférieur-hydroximino, Y signifie l'oxygène ou le groupe NH, n signifie 0 ou 1 et $R_{10}$ signifie un reste hydrocarboné aliphatique ayant 5-22 atomes de carbone, signifie un reste phényle ou naphtyle qui est à chaque fois non substitué ou substitué par un groupe cyano, alkyle inférieur, hydroxy-alkyle inférieur, amino-alkyle inférieur, (4-méthyl-pipérazinyl)-alkyle inférieur, trifluoro-méthyle, hydroxy, alcoxy inférieur, alcanoyloxy inférieur, un halogène, un groupe amino, alkyl inférieur-amino, Di-alkyl inférieur-amino, alcanoyl inférieur-amino, benzoylamino, carboxy ou par un groupe alcoxy inférieur-carbonyle, signifie un groupe phényl-alkyle inférieur, où le reste phényle est non substitué ou substitué comme indiqué plus haut, signifie un reste cycloalkyle ou cycloalcényle ayant jusqu'à 30 atomes de carbone, un groupe cycloalkyl-alkyle inférieur ou cycloalcénylalkyle inférieur ayant à chaque fois jusqu'à 30 atomes de carbone dans la partie cycloalkyle ou cycloalcényle, signifie un reste monocyclique ayant 5 ou 6 chaînons dans le cycle et 1-3 atomes dans le cycle, choisi parmi l'azote, l'oxygène et le soufre, sur lequel un ou deux restes benzène peuvent être condensés, ou signifie un groupe alkyle inférieur qui est substitué par un tel reste monocyclique, et les autres restes $R_4$, $R_5$, $R_6$, $R_7$ et $R_8$ indépendamment les uns des autres, signifient chacun l'hydrogène, un groupe alkyle inférieur qui est non substitué ou substitué par un groupe amino, alkyl inférieur-amino, di-alkyl inférieur-amino, pipérazinyle, pipéridinyle, pyrrolidinyle ou par un groupe morpholinyle, alcanoyle inférieur, trifluorométhyle, hydroxy, alcoxy inférieur, alcanoyloxy inférieur, un halogène, un groupe amino, alkyl inférieur-amino, dialkyl inférieur-amino, alcanoyl inférieur-amino, benzoylamino, carboxy ou alcoxy inférieur-carbonyle, et les sels de tels composés avec au moins un groupe formant un sel.

**4.** Les composés de formule I selon la revendication 1, où $R_1$ signifie un groupe pyridyle lié à un atome de carbone du cycle, qui est non substitué sur l'azote ou substitué par de l'oxygène, $R_2$ et $R_3$ signifient chacun l'hydrogène, $R_4$ signifie l'hydrogène ou un groupe alkyle inférieur, $R_5$ signifie l'hydrogène, un groupe alkyle inférieur ou alcoxy inférieur substitué par du fluor, $R_6$ signifie l'hydrogène, $R_7$ signifie un groupe nitro, alcoxy inférieur substitué par du fluor ou un reste de formule II, où $R_9$ signifie l'hydrogène, X signifie un groupe oxo, n signifie 0 et $R_{10}$ signifie un reste hydrocarboné aliphatique ayant 5-22 atomes de carbone, signifie un reste phényle qui est non substitué ou substitué par un groupe cyano, alkyle inférieur, (4-méthyl-pipérazinyl)-alkyle inférieur, alcoxy inférieur, un halogène ou par un groupe carboxy, signifie un reste cycloalkyle ayant jusqu'à 30 atomes de carbone ou un reste monocyclique ayant 5 ou 6 chaînons dans le cycle et 1-3 atomes de soufre dans le cycle, et $R_8$ signifie l'hydrogène et les sels pharmaceutiquement acceptables de tels composés avec au moins un groupe formant un sel.

**5.** Les composés de formule I selon la revendication 1, où $R_1$ signifie à chaque fois un groupe pyridyle lié à un atome de carbone ou un groupe N-oxidopyridyle, $R_2$ et $R_3$ signifient chacun l'hydrogène, $R_4$ signifie l'hydrogène ou un groupe alkyle inférieur, $R_5$ signifie l'hydrogène, un groupe alkyle inférieur ou trifluorométhyle, $R_6$ signifie l'hydrogène, $R_7$ signifie un groupe nitro, alcoxy inférieur substitué par du fluor ou un reste de formule II, où $R_9$ signifie l'hydrogène, X signifie oxo, n signifie le nombre 0 et $R_{10}$ signifie un groupe pyridyle lié à un atome de carbone, phényle non substitué ou substitué par un halogène, un groupe cyano, alcoxy inférieur, carboxy, alkyle inférieur ou 4-méthyl-pipérazinyl-méthyle, signifie un groupe $C_5$-$C_7$-alkyle, thiényle, 2-Naphtyle ou cyclohexyle, et $R_8$ signifie l'hydrogène et les sels pharmaceutiquement acceptables de tels composés avec au moins un groupe formant un sel.

**6.** Les composés selon l'une quelconque des revendications 1-5 de formule I, où $R_4$ et $R_8$ signifient chacun l'hydrogène et les autres substituants ont les significations indiquées dans une revendication précédente, et les sels pharmaceutiquement acceptables de tels composés avec au moins un groupe formant un sel.

**7.** Les composés selon l'une quelconque des revendications 1-5 de formule I, où au moins un des restes $R_4$ et $R_8$ signifient un groupe alkyle inférieur et les autres substituants ont les significations indiquées dans une revendication précédente, et les sels pharmaceutiquement acceptables de tels composés avec au moins un groupe formant un sel.

**8.** Les composés selon la revendication 1 de formule I, où $R_1$ signifie un groupe pyridyle lié à un atome de carbone, $R_2$, $R_3$, $R_4$, $R_5$, $R_6$ et $R_8$ signifient chacun l'hydrogène, et $R_7$ signifie un groupe nitro ou un reste de formule II, où $R_9$ signifie l'hydrogène, X signifie oxo, n signifie le nombre 0 et $R_{10}$ signifie un groupe pyridyle lié à un atome de carbone, signifie un groupe phényle non substitué ou substitué par le fluor, le chlore, un groupe cyano, alcoxy inférieur, carboxy, alkyle inférieur ou 4-méthyl-pipérazinyl-méthyle, signifie un groupe $C_5$-$C_7$-alkyle, thiényle ou cyclohexyle, et leurs sels pharmaceutiquement acceptables.

**9.** Un composé selon la revendication 1 de formule I, choisi parmi

la N-(3-nitro-phényl)-4-(3-pyridyl)-2-pyrimidine-amine,
la N-[3-(4-chlorobenzoylamido)-phényl]-4-(3-pyridyl)-2-pyrimidine-amine,
la N-(3-benzoylamido-phényl)-4-(3-pyridyl)-2-pyrimidine-amine,
la N-[3-(2-pyridyl)carboxamido-phényl]-4-(3-pyridyl)-2-pyrimidine-amine,
la N-[3-(3-pyridyl)carboxamido-phényl]-4-(3-pyridyl)-2-pyrimidine-amine,
la N-[3-(4-pyridyl)carboxamido-phényl]-4-(3-pyridyl)-2-pyrimidine-amine,
la N-(3-pentafluoro-benzoylamido-phényl)-4-(3-pyridyl)-2-pyrimidine-amine,
la N-[3-(2-carboxy-benzoylamido)-phényl]-4-(3-pyridyl)-2-pyrimidine-amine,
la N-(3-n-hexanoylamido-phényl)-4-(3-pyridyl)-2-pyrimidine-amine,
la N-(3-nitro-phényl)-4-(2-pyridyl)-2-pyrimidine-amine,
la N-(3-nitro-phényl)-4-(4-pyridyl)-2-pyrimidine-amine,
la N-[3-(2-méthoxy-benzoylamido)-phényl]-4-(3-pyridyl)-2-pyrimidine-amine,
la N-[3-(4-fluoro-benzoylamido)-phényl]-4-(3-pyridyl)-2-pyrimidine-amine,
la N-[3-(4-cyano-benzoylamido)-phényl]-4-(3-pyridyl)-2-pyrimidine-amine,
la N-[3-(2-thiénylcarboxamido)-phényl]-4-(3-pyridyl)-2-pyrimidine-amine,
la N-(3-cyclohexylcarboxamido-phényl)-4-(3-pyridyl)-2-pyrimidine-amine,
la N-[3-(4-méthyl-benzoylamido)-phényl]-4-(3-pyridyl)-2-pyrimidine-amine,
la N-[3-(4-chloro-benzoylamido)-phényl]-4-(4-pyridyl)-2-pyrimidine-amine,
la N-{3-[4-(4-méthyl-pipérazinométhyl)-benzoylamido]-phényl}-4-(3-pyridyl)-2-pyrimidine-amine,
la N-[5-(4-méthyl-benzoylamido)-2-méthyl-phényl]-4-(3-pyridyl)-2-pyrimidine-amine,
la N-[5-(2-naphtoylamido)-2-méthyl-phényl]-4-(3-pyridyl)-2-pyrimidine-amine,
la N-[5-(4-chloro-benzoylamido)-2-méthyl-phényl]-4-(3-pyridyl)-2-pyrimidine-amine,
la N-[5-(2-méthoxy-benzoylamido)-2-méthyl-phényl]-4-(3-pyridyl)-2-pyrimidine-amine,
la N-(3-trifluorométhoxy-phényl)]-4-(3-pyridyl)-2-pyrimidine-amine,
la N-(3-[1,1,2,2-tétrafluoro-éthoxy]-phényl)-4-(3-pyridyl)-2-pyrimidine-amine,
la N-(3-nitro-5-méthyl-phényl)-4-(3-pyridyl)-2-pyrimidine-amine,
la N-(3-nitro-5-trifluorométhyl-phényl)-4-(3-pyridyl)-2-pyrimidine-amine,
la N-(3-nitro-phényl)-4-(N-oxydo-3-pyridyl)-2-pyrimidine-amine,
la N-(3-benzoylamido-5-méthyl-phényl)-4-(N-oxydo-3-pyridyl)-2-pyrimidine-amine et les sels pharmaceutiquement acceptables de ces composés avec au moins un groupe formant un sel.

**10.** La N-(5-benzoylamido-2-méthyl-phényl)-4-(3- pyridyl)-2-pyrimidine-amine de formule I selon la revendication 1.

**11.** La N-{5-[4-(4-méthyl-pipérazino-méthyl)-benzoylamido]-2-méthyl-phényl}-4-(3-pyridyl)-2-pyrimidine-amine de formule I selon la revendication 1 ou un de ses sels d'addition d'acides pharmaceutiquement acceptables.

**12.** Un composé de formule I selon l'une quelconque des revendications 1-11 ou un sel pharmaceutiquement acceptable d'un tel composé avec au moins un groupe formant un sel pour une utilisation dans un procédé de traitement thérapeutique du corps humain ou animal.

**13.** Une composition pharmaceutique contenant un composé de formule I selon l'une quelconque des revendications 1-11 ou un sel pharmaceutiquement acceptable d'un tel composé avec au moins un groupe formant un sel, ensemble avec un véhicule pharmaceutique.

**14.** Utilisation d'un composé de formule I selon l'une quelconque des revendications 1-11 ou d'un sel pharmaceutiquement acceptable d'un tel composé avec au moins un groupe formant un sel, pour la préparation de compositions pharmaceutiques pour la chimiothérapie de tumeurs.

**15.** Utilisation d'un composé de formule I selon l'une quelconque des revendications 1-11 ou d'un sel pharmaceutiquement acceptable d'un tel composé avec au moins un groupe formant un sel, pour la préparation de compositions pharmaceutiques pour le traitement de l'athérosclérose.

**16.** Un procédé de préparation d'un composé de formule I selon la revendication 1 ou d'un sel d'un tel composé avec au moins un groupe formant un sel, caractérisé en ce qu'on fait réagir

a) un composé de formule III,

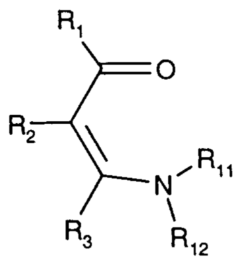

(III)

où $R_{11}$ et $R_{12}$ indépendamment l'un de l'autre, signifient chacun un groupe alkyle inférieur et $R_1$, $R_2$ et $R_3$ ont les significations données plus haut, où, dans un composé de formule III, les groupes fonctionnels présents, à l'exception des groupes participant à la réaction, se présentent, si nécessaire, sous forme protégée, ou un sel d'un tel composé avec un composé de formule IV,

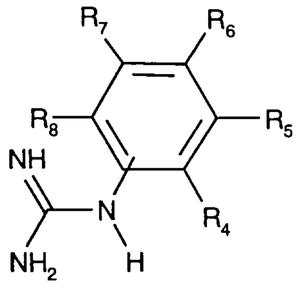

(IV)

où les substituants ont les significations données plus haut, où, dans un composé de formule IV, les groupes fonctionnels présents, à l'exception du groupe guanidino participant à la réaction, se présentent, si nécessaire, sous forme protégée, ou avec un sel d'un tel composé, et on élimine les groupes protecteurs présents, ou bien

b) pour la préparation d'un composé de formule I, où les restes $R_4$, $R_5$, $R_6$, $R_7$ et $R_8$ ont les significations données plus haut, à l'exception du groupe nitro et du groupe alcoxy inférieur substitué par du fluor, on fait réagir un composé de formule V,

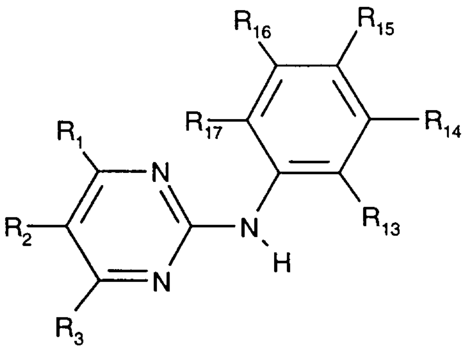

(V)

où un ou deux des restes $R_{13}$, $R_{14}$, $R_{15}$, $R_{16}$ et $R_{17}$ signifient chacun un groupe $R_9$-amino et les autres restes $R_{13}$, $R_{14}$, $R_{15}$, $R_{16}$ et $R_{17}$, indépendamment les uns des autres, signifient chacun l'hydrogène, un groupe alkyle inférieur qui est non substitué ou substitué par un groupe amino, alkyl inférieur-amino, di-alkyl inférieur-amino, pipérazinyle, pipéridinyle, pyrrolidinyle ou morpholinyle, signifient alcanoyle inférieur, trifluorométhyle, hydroxy, alcoxy inférieur, alcanoyloxy inférieur, un halogène, un groupe amino, alkyl inférieur-amino, di-alkyl inférieur-amino, alcanoyl inférieur-amino, benzoylamino, carboxy ou alcoxy inférieur-carbonyle et $R_1$, $R_2$, $R_3$ et $R_9$ ont les significations données plus haut, où, dans un composé de formule V, les groupes fonctionnels présents, à l'exception du ou des groupes $R_9$-amino participant à la réaction, se présentent, si nécessaire, sous forme protégée, avec un composé de formule VI,

$$HO\text{-}C(=X)\text{-}(Y)_n\text{-}R_{10} \qquad \text{(VI)}$$

où les substituants et les symboles ont les significations données plus haut, où, dans un composé de formule VI, les groupes fonctionnels présents, à l'exception du groupe HO-C(=X) participant à la réaction, se présentent, si nécessaire, sous forme protégée, ou avec un dérivé réactif d'un composé de formule VI, et on élimine les groupes protecteurs présents, ou bien

c) pour la préparation d'un composé de formule I, où $R_1$ signifie un groupe pyridyle, qui est substitué sur l'azote par de l'oxygène et où les autres substituants et symboles ont les significations données plus haut, on transforme un composé de formule I, où $R_1$ signifie un groupe pyridyle, avec un agent d'oxydation approprié, en composé N-oxido,
et, éventuellement, on transforme un composé de formule I obtenu selon l'un des procédés a à c en son sel, ou bien on transforme un sel obtenu d'un composé de formule I en composé libre.